# FASCICULE DE BREVET EUROPEEN

(11) **EP 1 844 761 B1**
(45) Date de publication et mention de la délivrance du brevet: **19.05.2010**
(21) Numéro de dépôt: 07103780.8
(22) Date de dépôt: 08.03.2007
(51) Int. Cl.: A61K 8/85, A61Q 3/02

(54) **Composition cosmétique ou pharmaceutique comprenant un polycondensat, procédé de traitement cosmétique employant ladite composition, ledit polycondensat et procédé de préparation**
Kosmetische oder pharmazeutische Zusammensetzung, die ein Polykondensat umfasst, kosmetisches Behandlungsverfahren, bei dem die besagte Zusammensetzung einsetzt wird, besagtes Polykondensat und Herstellungsverfahren
Cosmetic or pharmaceutical composition comprising a condensation polymer, cosmetic treatment method employing said composition, said condensation polymer and preparation method

(30) Priorité: 04.04.2006 FR 0651190
(43) Date de publication de la demande: 17.10.2007
(73) Titulaire: L'Oréal, 75008 Paris (FR)
(72) Inventeur: Rodriguez, Ivan, 60290, Cauffry (FR); Ramin, Roland, 75014, Paris (FR); Giustiniani, Pascal, 92250 La Garenne Colombes (FR); Malle, Gérard, 77580, Villiers S/Morin (FR); Ilekti, Philippe, 94700, Maison-Alfort (FR)
(74) Mandataire: Dodin, Catherine

(56) Documents cités:
- US-A- 2 915 488
- DATABASE WPI Week 198312 Derwent Publications Ltd., London, GB; AN 1983-28350K XP002409235 & JP 58 023614 A (ASANUMA KOGYO KK) 12 février 1983 (1983-02-12)
- DATABASE WPI Week 197910 Derwent Publications Ltd., London, GB; AN 1979-18769B XP002409236 & JP 54 011244 A (KANEBO LTD) 27 janvier 1979 (1979-01-27)
- DATABASE WPI Week 197814 Derwent Publications Ltd., London, GB; AN 1978-26140A XP002409237 & JP 53 018742 A (KANEBO LTD) 21 février 1978 (1978-02-21)
- DATABASE WPI Week 198808 Derwent Publications Ltd., London, GB; AN 1988-052800 XP002409238 & JP 63 008318 A (KOBAYASHI KOSE KK) 14 janvier 1988 (1988-01-14)

## Description

La présente invention a trait à de nouveaux polymères de la famille des polycondensats de type alkyde modifié, ainsi qu'à leur utilisation dans les compositions cosmétiques, notamment dans les vernis à ongles, aux compositions cosmétiques les contenant et aux procédés de préparation desdits polycondensats.

Les compositions cosmétiques filmogènes et notamment les compositions de vernis à ongles, doivent présenter un certain nombre de caractéristiques, qui permettent leur application et leur bonne tenue sur le support.
Notamment, les compositions cosmétiques présentent de préférence une bonne applicabilité et une bonne couvrance; une bonne adhérence sur le support (surface de l'ongle, cheveux); une certaine flexibilité et une bonne résistance du film en vue d'éviter les craquelures et l'écaillement dans le cas des vernis; la possibilité d'obtenir un film homogène brillant.

Dans le domaine des vernis à ongles, on utilise, à l'heure actuelle, comme matière filmogène principale une résine filmogène dite "résine primaire" qui est généralement la nitrocellulose. Il est également possible de la remplacer, en tout ou partie, par une résine polyvinylique telle que le butyrate de polyvinyle ou bien encore par de l'acétobutyrate ou de l'acétopropionate de cellulose.

Pour conférer une bonne adhérence, et ainsi garantir une bonne tenue dans le temps, on utilise également des résines secondaires de différentes natures, telles que des résines aryl-sulfonamide-formaldéhyde ou aryl-sulfonamide-époxy, des résines polyesters, des résines de type alkyde, des résines polyuréthannes, des résines polyester-polyuréthannes, des résines polyéther-polyuréthannes, des résines vinyliques et/ou acryliques, seules ou en mélange.
Ces résines secondaires permettent d'augmenter le pouvoir filmogène de la nitrocellulose et améliorent le brillant ainsi que l'adhérence des films.

Par ailleurs, pour régler la flexibilité du film sans affaiblir sa résistance physique, on utilise des agents plastifiants, tels que par exemple des phtalates ou des citrates.

Afin d'améliorer la tenue du film et sa résistance à l'écaillement, différentes résines secondaires, notamment de type alkyde modifiées, ont été proposées.
On peut en particulier citer le document FR2562793 qui décrit l'utilisation de benzoate de sucrose en association avec des résines de type toluène sulfonamide formaldéhyde; ou le document JP61246113 qui décrit l'utilisation de benzoate de sucrose en association avec une résine alkyde modifiée glycidyl versatate ester.
On peut aussi citer W02002243676 qui décrit l'utilisation d'une résine polyester néopentyl glycol trimellitate adipate en association avec des copolymères d'acrylates et de méthacrylates d'alkyle.
On connaît encore JP58023614 qui décrit l'utilisation d'un polyester modifié obtenu par condensation du pentaérythritol avec de l'acide cis-4-cyclohexene-1,2-dicarboxylique et des acides gras d'huile de ricin puis réaction avec un composé dioxirane de type résine époxy; ou encore JP54011244 qui décrit l'utilisation d'un polyester modifié obtenu par condensation du dipentaérythritol avec de l'acide cyclohexane-1,2-dicarboxylique et des acides gras d'huile de ricin puis réaction avec un composé dioxirane de type résine époxy.
Toutefois, ces associations, même si elles améliorent la tenue de façon significative, sont encore jugées insuffisantes dans une optique de longue tenue.

Le but de la présente invention est de proposer de nouveaux polymères qui peuvent être employés notamment comme résine secondaire et ainsi permettre d'améliorer significativement la tenue d'un dépôt filmogène, notamment dans un vernis à ongles, tout en lui conférant une excellente tenue dans le temps.

A cette fin, la demanderesse a recherché de nouveaux polycondensats de type alkyde, ayant les propriétés recherchées.
Les résines alkydes constituent une classe particulière de polyesters en étant le produit de réaction de polyols et d'acides polycarboxyliques, généralement modifié par des acides gras insaturés, tels que l'acide oléique, ou par des huiles insaturées, huile de soja ou de ricin par exemple, qui permettent de moduler leurs propriétés filmogènes, notamment leur vitesse de séchage, leur dureté, leur résistance, etc.
Ainsi, il a été proposé dans le document US2915488 des résines alkydes modifiées dans lesquelles une partie des acides gras provenant de l'huile de soja a été remplacée par de l'acide benzoïque. Ces nouvelles résines présentent des propriétés améliorées en terme de résistance aux alcalis et aux détergents; les films les contenant sèchent plus rapidement et sont plus durs. Toutefois, aucune application, notamment cosmétique ou topique, n'était envisagée pour ces résines.
Par ailleurs, on sait que les acides gras insaturés peuvent subir, au cours du temps, une autooxydation à l'origine de phénomènes de rancissement, qui peuvent donc déboucher sur des problèmes de conservation des compositions comprenant ces matières premières. Or, il est connu que les acides gras présents dans l'huile de soja sont majoritairement constitués de deux acides gras insaturés: environ 55% d'acide linoléique (C18:2) et 28% d'acide oléique (C18:1), selon "Surface Coatings Science and Technology", 2ème édition, JOHN WILEY & Sons, pages 104 et 105. Les résines modifiées décrites dans US2915488, qui comprennent une proportion élevée d'acides gras insaturés, présentent donc encore des inconvénients pour une utilisation en cosmétique.

Après d'importantes recherches, la demanderesse a découvert de façon surprenante et inattendue que certains polycondensats à haute teneur en acides carboxyliques particuliers, dont les acides aromatiques, pouvaient conduire à des performances améliorées en terme de brillance et de longue tenue du film obtenu.

La présente invention a donc pour objet une composition cosmétique ou pharmaceutique comprenant, dans un milieu cosmétiquement ou pharmaceutiquement acceptable, au moins un polycondensat susceptible d'être obtenu par réaction :
- de 15 à 30%, en poids, par rapport au poids total du polycondensat, d'au moins un polyol hydrocarboné saturé, linéaire ou ramifié, comprenant 3 à 18 atomes de carbone, et 3 à 6 groupes hydroxyles (OH);
- de 5 à 40% en poids, par rapport au poids total du polycondensat, d'au moins un acide monocarboxylique non aromatique de formule RCOOH, dans laquelle R est un radical hydrocarbone saturé, linéaire, ramifié et/ou cyclique, comprenant 5 à 31 atomes de carbone;
- de 10 à 55% en poids, par rapport au poids total du polycondensat, d'au moins un acide monocarboxylique aromatique de formule R'COOH, dans laquelle R' est un radical benzoïque ou naphtoïque, éventuellement substitué par 1 à 3 radicaux alkyles, saturés ou insaturé, linéaire, ramifiés et/ou cycliques, comprenant 1 à 32 atomes de carbone.
- de 10 à 25% en poids, par rapport au poids total du polycondensat, d'au moins un acide polycarboxylique comprenant 2 à 4 groupes carboxyliques COOH, choisi parmi les acides polycarboxyliques aliphatiques saturés, linéaires, comprenant 2 à 20 atomes de carbone: et les acides polycarboxyliques aromatiques comprenant 8 à 12 atomes de carbone;
et/ou un anhydride cyclique choisi parmi l'anhydride phtalique, l'anhydride trimellitique, l'anhydride maleïque et l'anhydride succinique.

Un autre objet de l'invention est un polycondensat susceptible d'être obtenu par réaction :
- d'au moins un polyol parmi, seul ou en mélange, le 1,2,6-hexanetriol, le triméthyloléthane, le triméthylolpropane, le glycérol; le pentaérythritol, létythritol, le diglycérol, le ditriméthylolpropane; le xylitol, le sorbitol, le mannitol, le dipentaérythritol et/ou le triglycérol, présent en une quantité de 15 à 30% en poids, par rapport au poids total du polycondensat final;
- d'au moins un acide monocarboxylique non aromatique choisi parmi, seul ou en mélange, l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptyiheptanoïque, l'acide 3,5,5-triméthylhexanoïque, l'acide octanoïque, l'acide isooctanoïque, l'acide nonanoïque, l'acide décanoïque, l'acide isononanoïque, l'acide laurique, l'acide tridécanoïque, l'acide myristique, l'acide palmitique l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentylpropionique, l'acide cyclohexanecarboxylique, l'acide cyclohexylacétique l'acide 4-cyclohexylbutyrique; présent en une quantité de 5 à 40% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque; présent en une quantité de 10 à 55% en poids, par rapport au poids total du polycondensat final, et
- d'au moins un acide polycarboxylique ou un de ses anhydrides, choisi parmi, seul ou en mélange, l'acide décanedioïque, l'acide dodécanedioïque, l'acide cyclopropanedicarboxylique, l'acide cyclohexanedicarboxylique, l'acide cyclobutanedicarboxylique, l'acide naphtaléne-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide, pimélique, l'acide sébacique, l'acide azélaïque, l'acide glutarique, l'acide adipique, l'acide fumarique, l'acide maléïque; l'acide cyclohexanetricarboxylique, l'acide trimellitique, l'acide 1,2,3-benzénecarboxylique, l'acide 1,3,5-benzènetricarboxylique; l'acide butanetétracarboxylique, l'acide pyroméllitique, l'anhydride phtalique, l'anhydride trimellitique, l'anhydride maléïque est l'anhydride succinique; présent en une quantité de 10 à 25% en poids, par rapport au poids total du polycondensat final.

Encore un autre objet de est un procédé de préparation desdits polycondensats consistant:
- à mélanger le polyol et les acides monocarboxyliques aromatiques et non aromatiques,
- à chauffer le mélange sous atmosphère inerte, d'abord jusqu'à la température de fusion, puis à une température comprise entre 150 et 220°C jusqu'à consommation complète des acides monocarboxyliques, puis
- à éventuellement refroidir le mélange à une température comprise entre 90 et 150°C.
- à ajouter l'acide polycarboxylique et/ou l'anhydride cyclique, et optionnellement la silicone à fonctions hydroxyles ou carboxyliques, puis
- à chauffer à nouveau à une inférieure ou égale à 220°C.

On a constaté que les nouveaux polycondensats branchés de type alkyde selon l'invention pouvaient permettre de formuler des composition de vernis à ongles de longue tenue est présentant une couvrance et une brillance améliorées par rapport à l'état de l'art.
Par ailleurs, ces polycondensats sont très solubles dans les solvants de type acétate de butyle ou d'éthyle, ce facilite leur mise en oeuvre dans le domaine cosmétrique, notamment dans les vernis a ongles.
Un autre avantage des polycondensats selon l'invention est qu'ils peuvent être préparés aisément, en une seule étape de synthèse, et sans produire de déchets, ceci à faible coût
Un autre avantage dans le fait qu'il est aisément possible de modifier la structure et/ou les propriétés des polycondensats selon invention, en faisant varier la nature chimique des différents constituants et/ou leurs proportions.

Les polycondensats selon l'invention sont avantageusement branchés (ramifies); on peut penser que ceci permet de générer un réseau par enchevêtrement des chaînes polymériques, est donc d'obtenir les propriétés recherchées, notamment en terme de tenue améliorée et en terme de solubilité. On a en effet constaté que les polycondensats linéaires ne permettraient pas d'obtenir une amélioration notable de la tenue de la composition, et que les polycondensats de type dendrimères, dont les chaînes sont réguliéres, ne présentaient pas une solubilité optimale.

Les polycondensats selon l'invention sont des polycondensats de type alkyde, et sont donc susceptibles d'être obtenus par estérification/polycondensation, selon les méthodes connues de l'homme du métier, des constituants décrits ci-après. L'un des constituants nécessaires pour la préparation des polycondensats selon l'invention est un compose comprenant 3 à 6 groupes hydroxyles (polyol), notamment 3 à 4 groupes hydroxyles. On peut bien évidemment utiliser un mélange de tels polyols.
Ledit polyol est un composé hydrocarboné saturé, linéaire ou ramifié, comprenant 3 à 18 atomes de carbone, notamment 3 à 12, voire 4 à 10 atomes de carbone, et 3 à 6 groupes hydroxy (OH).

Il peut être choisi parmi, seul ou en mélange :
- les triols, tels que le 1,2,6-hexanetriol, le triméthyloléthane, le triméthylolpropane, le glycérol;
- les tétraols, tels que le pentaérythritol (tétraméthylolméthane), l'érythritol, le diglycérol ou le ditriméthylolpropane;
- les pentols tels que le xylitol,
- les hexols que le sorbitol et le mannitol; ou encore le dipentaérythritol ou le triglycérol.
De préférence, le polyol est choisi parmi le glycérol le pentaérythritol, le sorbitol et leurs mélanges; et encore est du pentaérythritol.

Le polyol, ou le mélange de polyol, représente de préférence 15 à 30% en poids, notamment. 16 a 28% en poids, et mieux 18 à 25% en poids, du poids total du polycondensat final.

Un autre constituant nécessaire pour la préparation des polycondensats selon l'invention est un acide monocarboxylique non aromatique de formule RCOOH, dans laquelle R est un radical hydrocarboné saturé, linéaire, ramifié et/ou cyclique, comprenant 5 à 31 atomes de carbone, notamment 7 à 27 atomes de carbone, et encore mieux 9 à 19 atomes de carbone, voire 11 à 17 atomes de carbone.
On peut bien évidemment utiliser un mélange de tels acides monocarboxyliques non aromatiques.
De préférence, le radical R est linéaire ou ramifié, préférentiellement en C5-C31.

Parmi les acides monocarboxyliques non aromatiques susceptibles d'être employés, on peut citer, seul ou en mélange,
- les acides monocarboxyliques saturés tels que l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide décanoïque, l'acide isononanoïque l'acide laurique, l'acide tridécanoïque, l'acide myristique, l'acide palmitique, l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentylpropionique, l'acide cyclohexanecarboxylique, l'acide cyclohexylacétique, l'acide 4-cyclohexylbutyrique.
De préférence, on peut utiliser l'acide 2-éthylhexanoïque, l'acide isooctanoïque, l'acide laurique, l'acide palmitique, l'acide isostéarique, et leurs mélanges, et encore mieux l'acide isostéarique seul.

Ledit acide monocarboxylique non aromatiques, ou le mélange desdits acides, représente de préférence 5 à 40% en poids, notamment 8 à 38% en poids, et mieux 10 à 35% en poids, du poids total du polycondensat, final,

Un autre constituant nécessaire pour la préparation des polycondensats selon l'invention est un acide aromatique comprenant 7 à 11 atomes de carbone, éventuellement en outre substitué par 1 a 3 radicaux alkyles, saturés ou insaturés, linéaires, ramifiés cycliques, qui comprennent 1 à 32 atomes de carbone, notamment 2 à 12, voire 3 à 8 atomes de carbone.
On peut bien évidemment utiliser un mélange de tels acides monocarboxyliques aromatiques.
Par acide monocarboxylique aromatique, on entend un composé de formule R'COOH. dans laquelle R' est un radical hydrocarboné aromatique, comprenant 6 à 10 atomes de carbone, et en les radicaux benzoïque et naphtoïque. Ledit radical R' peut en outre être substitué par 1 à 3 radicaux alkyles, saturés ou insaturés, linéaires, ramifiés et/ou cycliques, comprenant 1 à 32 atomes de carbone, notamment 2 à 12, voire 3 à 8 atomes de carbone; et notamment choisis parmi méthyle, éthyle, propyle, isopropyle, butyle, isobutyle, terbutyle, pentyle, isopentyle, néopentyte, cyclopentyle, hexyle, cyclohexyle, heptyle, isoheptyle, octyle ou isooctyle.

Parmi les acides monocarboxyliques aromatiques susceptibles d'être employés, on peut citer, seul ou en l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque.
De préférence, on peut utiliser l'acide benzoïque, l'acide o-toluique, l'acide m-toluique, l'acide 1-naphtoïque, seuls ou en mélanges; et encore mieux l'acide benzoïque seul.

Ledit acide aromatique, ou le mélange desdits acides, représente de préférence 18 à 55% en poids, notamment 20 à 52% en poids, voire 22 à 52% en poids, et mieux 25 à 50% en poids, du poids total du polycondensat final.

Un autre constituant pour la préparation des polycondensats selon l'invention est un acide polycarboxylique, saturé ou insaturé, voire aromatique, linéaire, ramifié et/ou comprenant 2 à 4 groupes carboxyliques COOH; et/ou un anhydride choisi parmi, l'anhydride phtalique, l'anhydride trimellitique, l'anhydride maléïque et l'anhydride succinique.

On peut bien évidemment utiliser un mélange de tels acides polycarboxyliques et/ou d'anhydrides.

Ledit acide polycarboxylique est choisi parmi les acides polycarboxyliques aliphatiques saturés, linéaires, comprenant 2 à 20 atomes de carbone, notamment 3 à 18 atomes de carbone, voire 4 à 12 atomes de carbone; et les acides polycarboxyliques aromatiques comprenant a à 12 atomes de carbone.

Parmi les acides polycarboxyliques et les anhydrides susceptibles d'être employés, on peut citer, seul on en mélange :
- les acides dicarboxyliques tels que l'acide décanedioïque, l'acide, dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide pimélique, l'acide sébacique, l'acide azélaïque, l'acide glutarique, l'acide adipique, l'acide fumarique, l'acide maléïque;
- les acides tricarboxyliques tels que l'acide cyclohexanetricarboxylique, l'acide trimellitique, l'acide 1,2,3-benzènetricarboxylique, l'acide 1,3,5-benzènetricarboxylique;
- les acides tels que l'acide butanetétracarboxylique et l'acide pyroméllitique,
- l'anhydride phtalique, l'anhydride trimellitique, l'anhydride maléïque et l'anhydride succinique.
De préférence, on peut utiliser l'anhydride phtalique et/ou l'acide isophtalique, et encore mieux l'acide isophtalique seul.

Ledit acide polycarboxylique et/ou son anhydride cyclique, représente de préférence 10 à 25% en poids, notamment 11 à 22% en poids,et mieux 12 à 20% en poids, du poids total du polycondensat final.

Le polycondensat selon l'invention peut en outre comprendre une silicone à fonction hydroxyle (OH) et/ou carboxylique (COOH).
Elle peut comprendre 1 à 3 fonctions hydroxyle et/ou carboxylique, et comprend de préférence deux fonctions hydroxyle ou bien deux fonctions carboxyliques. Ces fonctions peuvent être situées en bout de chaîne ou dans la chaîne, mais avantageusement en bout de chaîne.
On emploie de préférence des silicone ayant une masse moléculaire moyenne en poids (Mw) comprise entre 300 et 20000, notamment 400 et 10 000, voire 800 et 4000.

Cette silicone peut être de formule : 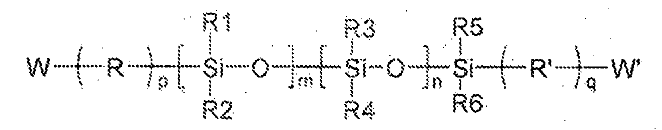 dans laquelle:
- W et W'sont, indépendamment l'un de l'autre, OH ou COOH; de préférence W=W';
- p et q sont, indépendamment l'un de l'autre, égaux à 0 ou 1,
- R et R' sont, indépendamment l'un de l'autre, un radical divalent carbone, notamment hydrocarbone, saturé ou insaturé, voire aromatique, linéaire, ramifié et/ou cyclique; comprenant 1 à 12 atomes de carbone, notamment 2 à 8 atomes de carbone, et comprenant éventuellement en outre 1 ou plusieurs hétéroatomes choisis parmi O, S et N, notamment O (ether);
   notamment R et/ou R' peuvent être de formule -(CH₂)ₐ- avec a=1-12, et notamment méthylène, éthylène, propyléne, phénylène;
   ou bien de formule -[(CH₂)ₓ O]_{z}- avec x = 1, 2 ou 3 et z = 1-10; en particulier x=2 ou 3 et z=1-4; et mieux x=3 et z=1.
- R1 à R6 sont, indépendamment l'un de l'autre, un radical carboné linéaire, ramifié et/ou cyclique, saturé ou insaturé voire aromatique; comprenant 1 à 20 atomes de carbone, notamment 2 à 12 atomes de carbone; de préférence, R1 à R6 sont saturés ou bien aromatiques, et peuvent notamment être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyles, en particulier le radical cyclohexyle, les radicaux aryles, notamment phényle et naphtyle, les radicaux arylalkyles, notamment benzyle et phényléthyle, ainsi que les radicaux tolyle et xylyle.
- m et n sont, indépendamment l'un de l'autre, des entiers compris entre 1 et 140, et sont tels que la masse moléculaire moyenne en poids (Mw) de la silicone est comprise entre 300 et 20 000, notamment entre 400 et 10 000, voire entre 800 et 4000.

On peut notamment citer les polyalkylsiloxanes α,ω-diol ou α,ω-dicarboxylique, et notamment les polydiméthysiloxanes α,ω-diol et les polydiméthylsiloxanes α,ω-dicarboxylique; les polyarylsiloxanes α,ω-diol ou α,ω-dicarboxylique et notamment les polyphénylsiloxanes α,ω-diol ou α,ω-dicarboxylique; les polyarylsiloxanes à fonctions silanol tels que le polyphénylsiloxane; les polyalkylsiloxanes à fonctions silanol tels que le polydiméthylsiloxane; les polyaryl/alkylsiloxanes à fonctions silanol tels que le polyphényl/méthylsiloxane ou encore le polyphényl/propylsiloxane.
Tout particulièrement, on utilisera les polydiméthysiloxanes α,ω-diol de masse moléculaire moyenne en poids (Mw) comprise entre 400 et 10 000, voire entre 500 et 5000, et notamment entre 800 et 4000.

Lorsqu'elle est présente, ladite silicone peut de préférence représenter 0,1 à 15% en poids, notamment 1 à 10% en poids, voire 2 à 8% en poids, du poids du polycondensat.

Dans un mode de réalisation préféré de l'invention, l'acide monocarboxylique aromatique est présent en quantité molaire supérieure ou égale à celle de l'acide monocarboxylique non aromatique; notamment le rapport entre le nombre de mole d'acide monocarboxylique aromatique et le nombre de mole d'acide monocarboxylique non aromatique est de préférence compris entre 1,2 et 8, en particulier entre 1,3 et 7,8, voire entre 1,4 et 7,5 et encore mieux entre 1,9 et 7.
On a constaté que cela permet notamment d'obtenir un polymère avantageusement soluble dans les esters dits courts (type acétate de butyle ou d'éthyle) généralement employés pour formuler des compositions cosmétiques de type vernis à ongles; par ailleurs, le film obtenu présente une rigidité adéquate pour son utilisation dans les formules de vernis à ongles.

De préférence, le polycondensat selon l'invention est susceptible d'être obtenu par réaction :
- d'au moins un polyol choisi parmi, seul ou en mélange, le 1,2,6-hexanetriol, le triméthyloléthane, le triméthylolpropane, le glycérol; le pentaérythritol, l'érythritol, le diglycérol, le ditriméthylolpropane; le xylitol, le sorbitol, le mannitol, le dipentaérythritol et/ou le triglycérol;
   présent de préférence en une quantité de 15 à 30% en poids, notamment 16 à 28% en poids, et mieux 18 à 25% en poids, par rapport au poids total du polycondensat final;
- d'au moins un acide monocarboxylique non aromatique choisi parmi, seul ou en mélange, l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide décanoïque, l'acide isononanoïque, l'acide laurique, l'acide tridécanoïque, l'acide myristique, l'acide palmitique, l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentylpropionique, l'acide cyclohexanecarboxylique, l'acide cyclohexylacétique, l'acide 4-cyclohexylbutyrique;
   présent de préférence en une quantité de 5 à 40% en poids, notamment 8 à 38% en poids, et mieux 10 à 35% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque;
   présent de préférence en une quantité de 10 à 55% en poids, notamment 20 à 52% en poids, et mieux 25 à 50% en poids, par rapport au poids total du polycondensat final; et
- d'au moins un acide polycarboxylique ou un de ses anhydrides, choisi parmi, seul ou en mélange, l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide pimélique, l'acide sébacique, l'acide azélaïque, l'acide glutarique, l'acide adipique, l'acide fumarique, l'acide maléïque; l'acide cyclohexanetricarboxylique, l'acide trimellitique, l'acide 1,2,3-benzènetricarboxylique, l'acide 1,3,5-benzènetricarboxylique; l'acide butanetétracarboxylique, l'acide pyroméllitique, l'anhydride phtalique, l'anhydride trimellitique, l'anhydride maléïque et l'anhydride succinique;
   présent de préférence en une quantité de 10 à 25% en poids, notamment 11 à 22% en poids, et mieux 12 à 20% en poids, par rapport au poids total du polycondensat final.

Préférentiellement, le polycondensat selon l'invention est susceptible d'être obtenu par réaction :
- d'au moins un polyol choisi parmi, seul ou en mélange, le glycérol, le pentaérythritol, le sorbitol et leurs mélanges, et encore mieux le pentaérythritol seul; présent en une quantité de 15 à 30% en poids, notamment 16 à 28% en poids, et mieux 18 à 25% en poids, par rapport au poids total du polycondensat final;
- d'au moins un acide monocarboxylique non aromatique choisi parmi, seul ou en mélange, l'acide 2-éthylhexanoïque, l'acide isooctanoïque, l'acide laurique, l'acide palmitique, l'acide isostéarique, et leurs mélanges, et encore mieux l'acide isostéarique seul; présent en une quantité de 5 à 40% en poids, notamment 8 à 38% en poids, et mieux 10 à 35% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide 1-naphtoïque, et encore mieux l'acide benzoïque seul; présent en une quantité de 10 à 55% en poids, notamment 20 à 52% en poids, et mieux 25 à 50% en poids, par rapport au poids total du polycondensat final; et
- d'au moins un acide polycarboxylique ou un de ses anhydrides, choisi parmi, seul ou en mélange, l'anhydride phtalique et l'acide isophtalique, et encore mieux l'acide isophtalique seul; présent en une quantité de 10 à 25% en poids, notamment 11 à 22% en poids, et mieux 12 à 20% en poids, par rapport au poids total du polycondensat final.

De préférence, le polycondensat selon l'invention présente :
- un indice d'acide, exprimé en mg d'hydroxyde de potassium par g de polycondensat, supérieur ou égal à 8; notamment compris entre 8 et 40, et encore mieux compris entre 10 et 30; et/ou
- un indice d'hydroxyle exprimé en mg d'hydroxyde de potassium par g de polycondensat, supérieur ou égal à 30; notamment compris entre 30 et 100, et encore mieux compris entre 40 et 90.
Ces indices d'acide et d'hydroxyle peuvent être aisément déterminés par l'homme du métier par les méthodes analytiques habituelles.

De préférence, le polycondensat selon l'invention présente une viscosité, mesurée à 110°C, comprise entre 75 et 6000 mPa.s, notamment entre 80 et 5500 mPa.s, voire entre 90 et 5000 mPa.s, et encore mieux entre 200 et 4800 mPa.s. Cette viscosité est mesurée de la manière décrite avant les exemples.

Par ailleurs, le polycondensat est avantageusement soluble dans les esters courts, comprenant au total 3 à 8 atomes de carbone, notamment les acétates d'acides carboxyliques en C1-C6, et en particulier l'acétate de butyle et/ou l'acétate d'éthyle.

Par soluble, on entend que le polymère forme une solution limpide dans l'acétate de butyle ou l'acétate d'éthyle, à raison d'au moins 50% en poids, à 25°C; de préférence, le polymère selon l'invention est soluble à raison d'au moins 70% en poids dans l'acétate de butyle ou l'acétate d'éthyle.

De préférence, la solution du polymère selon l'invention dans l'acétate de butyle ou l'acétate d'éthyle, à 25°C, à une concentration de 70% en poids, présente une viscosité comprise entre 100 et 1500 mPa.s, notamment entre 120 et 900 mPa.s. La méthode de mesure est donnée avant les exemples.

Le polycondensat selon l'invention peut être préparé par les procédés d'estérification/polycondensation usuellement employés par l'homme du métier. A titre d'illustration, un procédé général de préparation consiste :
- à mélanger le polyol et les acides monocarboxyliques aromatiques et non aromatiques,
- à chauffer le mélange sous atmosphère inerte, d'abord jusqu'à la température de fusion (généralement 100-130°C) et ensuite à une température comprise entre 150 et 220°C jusqu'à consommation complète des acides monocarboxyliques (atteint lorsque l'indice d'acide est inférieur ou égal à 1), de préférence en distillant au fur et à mesure l'eau formée, puis
- à éventuellement refroidir le mélange à une température comprise entre 90 et 150°C,
- à ajouter l'acide polycarboxylique et/ou l'anhydride cyclique, et optionnellement la silicone à fonctions hydroxyles ou carboxyliques, en une seule fois ou de façon séquencée, puis
- à chauffer à nouveau à une température inférieure ou égale à 220°C, notamment comprise entre 170 et 220°C, de préférence en continuant à éliminer l'eau formée, jusqu'à l'obtention des caractéristiques requises en terme d'indice d'acide, de viscosité, d'indice d'hydroxyle et de solubilité.

Il est possible d'ajouter des catalyseurs d'estérification conventionnels, par exemple de type acide sulfonique (notamment à une concentration pondérale comprise entre 1 et 10%) ou type titanate (notamment à une concentration pondérale comprise entre 5 et 100 ppm).
Il est également possible de réaliser la réaction, en tout ou en partie, dans un solvant inerte tel que le xylène et/ou sous une pression réduite, pour faciliter l'élimination de l'eau.
Avantageusement, on n'utilise ni catalyseur ni solvant.

Ledit procédé de préparation peut comprendre en outre une étape d'addition d'au moins un agent antioxydant dans le milieu réactionnel, notamment à une concentration pondérale comprise entre 0,01 et 1%, par rapport au poids total de monomères, de façon à limiter les éventuelles dégradations liées à un chauffage prolongé.
L'agent antioxydant peut être de type primaire ou de type secondaire, et peut être choisi parmi les phénols encombrés, les amines secondaires aromatiques, les composés organophosphorés, les composés soufrés, les lactones, les bisphénols acrylés; et leurs mélanges.
Parmi les antioxydants particulièrement préférés, on peut notamment citer le BHT, le BHA , le TBHQ, le 1,3,5-trimethyl-2,4,6,tris(3,5-di-tertbutyl-4-hydroxybenzyl)-benzène, l'octadecyl-3,5,di-tertbutyl-4-hydroxycinnamate, le tetrakis-methylene-3-(3,5-di-tertbutyl-4-hydroxy-phenyl)propionate méthane, l'octadecyl-3-(3,5-di-tertbutyl-4-hydroxyphenyl)propionate 2,5-di-tertbutyl hydroquinone, le 2,2-methyl-bis-(4-methyl-6-tertbutyl phénol), le 2,2-methylene-bis-(4-ethyl-6-tertbutyl phénol), le 4,4-butylidene-bis(6-tertbutyl-m-cresol), le N,N-hexamethylene bis(3,5-di-tertbutyl-4-hydroxyhydrocinnamamide), le pentaerythritol tetrakis (3-(3,5-di-tertbutyl-4-hydroxyphenyl)propionate) notamment celui commercialisé par CIBA sous le nom IRGANOX 1010; l'octadecyl 3-(3,5-di-tertbutyl-4-hydroxphenyl) propionate notamment celui commercialisé par CIBA sous le nom IRGANOX 1076; la 1,3,5-tris(3,5-di-tertbutyl-4-hydroxybenzyl)-1,3,5-triazine-2,4,6(1H,3H,5H)trione notamment celui commercialisée par Mayzo of Norcross, Ga sous le nom BNX 3114; le di(stearyl)pentaerythritol diphosphite, le tris(2,4-ditertbutyl phenyl)phosphite notamment celui commercialisé par CIBA sous le nom IRGAFOS 168; le dilauryl thiodipropionate notamment celui commercialisé par CIBA sous le nom IRGANOX PS800; le bis(2,4-di-tertbutyl)pentaerythritol diphosphite notamment celui commercialisé par CIBA sous le nom IRGAFOS 126; le bis(2,4-bis)[2-phénylpropan-2-yl]phényl)pentaérythritol diphosphite, le triphénylphosphite, le (2,4-di-tertbutylphenyl)pentaerythritol diphosphite notamment celui commercialisé par GE Specialty Chemicals sous le nom UL TRANOX 626; le tris(nonylphenyl)phosphite notamment celui commercialisé par CIBA sous le nom IRGAFOS TNPP; le mélange 1:1 de N,N-hexamethylenebis(3,5-di-tertbutyl-4-hydroxy-hydrocinnamamide) et de tris(2,4-di-tertbutylphenyl)phosphate notamment celui commercialisé par CIBA sous le nom Irganox B 1171; le tétrakis (2,4-di-tert-butylphényl)phosphite notamment celui commercialisé par CIBA sous le nom IRGAFOS P-EPQ; le distéarylthiodipropionate notamment celui commercialisé par CIBA sous le nom IRGANOX PS802; le 2,4-bis(octylthiométhyl)o-crésol notamment celui commercialisé par CIBA sous le nom IRGANOX 1520; le 4,6-bis(dodécylthiométhyl)o-crésol notamment celui commercialisé par CIBA sous le nom IRGANOX 1726.

Les polycondensats selon l'invention peuvent être utilisés très avantageusement dans une composition notamment cosmétique ou pharmaceutique, qui comprend par ailleurs un milieu physiologiquement, notamment cosmétiquement ou pharmaceutiquement, acceptable, c'est-à-dire un milieu compatible avec les tissus cutanés comme la peau du visage ou du corps, et les matières kératiniques telles que les cheveux, les cils, les sourcils et les ongles.
La quantité de polycondensat présente dans les compositions dépend bien entendu du type de composition et des propriétés recherchées et peut varier à l'intérieur d'une gamme très large, comprise généralement entre 0,1 et 70% en poids, de préférence entre 2 et 50% en poids, notamment entre 3 et 35% en poids, voire entre 5 et 20% en poids, et mieux entre 6 et 18% en poids, par rapport au poids de la composition cosmétique ou pharmaceutique finale.

La composition peut alors comprendre, selon l'application envisagée, les constituants habituels à ce type de composition.
La composition selon l'invention peut avantageusement comprendre un milieu solvant des polymères selon l'invention, qui peut comprendre au moins un composé choisi parmi l'eau, les alcools, les polyols, les cétones, les esters, les éthers, les alcanes, les aldéhydes, les huiles carbonées, les huiles de silicone, les huiles de silicone fluorées, et leurs mélanges; de préférence un milieu solvant organique comprenant un solvant organique ou un mélange de solvants organiques..

De préférence, le milieu physiologiquement acceptable de la composition selon l'invention peut comprendre au moins un solvant organique choisi parmi :
- les cétones liquides à température ambiante (25°C) tels que la méthyléthylcétone, la méthylisobutylcétone, la diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyléther de propylène glycol, le mono n-butyl éther de dipropylène glycol ;
- les éthers cycliques tels que la gamma-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyle, l'acétate de n-butyle, l'acétate d'isopentyle, l'acétate de méthoxypropyle, le lactate de butyle ;
- les éthers liquides à température ambiante tels que le diéthyléther, le diméthylé-ther ou le dichlorodiéthyléther ;
- les alcanes liquides à température ambiante, notamment en C5-C12, tels que le décane, l'heptane, le dodécane, l'isododécane, le cyclohexane ;
- les aldéhydes liquides à température ambiante tels que le benzaldéhyde, l'acé-taldéhyde ;
- et leurs mélanges.
De préférence, le solvant est choisi parmi les esters à chaîne courte ayant de 3 à 8 atomes de carbone, tels que l'acétate d'éthyle, l'acétate de méthyle, l'acétate de propyle, l'acétate d'isopropyle, l'acétate de n-butyle, l'acétate d'isopentyle, l'acétate de méthoxypropyle, le lactate de butyle; les alcools liquides à température ambiante tels que l'éthanol, l'isopropanol, le diacétone alcool, le 2-butoxyéthanol, le cyclohexanol; et leurs mélanges.

Le solvant organique, seul ou en mélange, peut représenter 10 à 95% en poids, par rapport au poids total de la composition, de préférence 15% à 80% en poids, et mieux 20 à 60% en poids.

La composition selon l'invention peut également comprendre des huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée. Parmi les huiles susceptibles d'être présentes dans la composition selon l'invention, on peut citer, seules ou en mélange, les huiles hydrocarbonées telles que l'huile de paraffine ou de vaseline; le perhydrosqualène; l'huile d'arara; l'huile d'amande douce, de calophyllum, de palme, de ricin, d'avocat, de jojoba, d'olive ou de germes de céréales; des esters d'acide lanolique, d'acide oléique, d'acide laurique, d'acide stéarique; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones. On peut également utiliser des huiles volatiles, telles que la cyclotétradiméthylsiloxane, la cyclopentadiméthylsiloxane, la cyclohexadiméthylsiloxane, le méthylhexyldiméthylsiloxane, l'hexaméthyldisiloxane ou les isoparaffines.
Les huiles, seules ou en mélange, peuvent représenter 0,01 à 20 % en poids, par rapport au poids total de la composition, de préférence 0,05 à 10% en poids, et mieux 0,1 à 8% en poids.

La composition selon l'invention peut comprendre avantageusement un polymère filmogène.
Selon la présente invention, on entend par "polymère filmogène", un polymère apte à former à lui seul ou en présence d'un agent auxiliaire de filmification, un film continu et adhérent sur un support, notamment sur les matières kératiniques.
Parmi les polymères filmogènes utilisables dans la composition de la présente invention, on peut citer les polymères synthétiques, de type radicalaire ou de type polycondensat, les polymères d'origine naturelle et leurs mélanges. Ils peuvent être choisis en particulier parmi les polymères cellulosiques tels que la nitrocellulose, l'acétate de cellulose, l'acétobutyrate de cellulose, l'acétopropionate de cellulose, l'éthyl cellulose, ou bien encore les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy ou encore les résines éthyl tosylamide.
Le polymère filmogène peut être présent dans la composition selon l'invention en une teneur allant de 1 à 70% en poids, par rapport au poids total de la composition, de préférence allant de 2 à 60% en poids, et mieux de 5 à 45% en poids.

Pour améliorer les propriétés filmogènes de la composition, notamment lorsqu'il s'agit d'un vernis à ongles, il est possible d'y ajouter un agent auxiliaire de filmification.
Un tel agent auxiliaire de filmification peut être choisi parmi tous les composés connus de l'homme du métier comme étant susceptibles de remplir la fonction recherchée, et être notamment choisi parmi les agents plastifiants et les agents de coalescence. En particulier, on peut citer, seuls ou en mélange, les plastifiants et agents de coalescence usuels, tels que :
- les glycols et leurs dérivés, tels que le diéthylène glycol éthyléther, le diéthylène glycol méthyléther, le diéthylène glycol butyléther, le diéthylène glycol hexyléther, l'éthylène glycol éthyléther, l'éthylène glycolbutyléther, l'éthylène glycol hexyléther;
- les polyéthylène glycols, les polypropylène glycols, les copolymères polyéthylène glycols-polypropylène glycols et leurs mélanges, notamment les polypropylène glycols de haut poids moléculaire, ayant par exemple une masse moléculaire allant de 500 à 15000;
- les esters de glycol ;
- les dérivés de propylène glycol et en particulier le propylène glycol phényléther, le propylène glycol diacétate, le dipropylène glycol éthyléther, le tripropylène glycol méthyléther et le diéthylène glycol méthyléther, le propylène glycol butyléther ;
- des esters d'acides, notamment carboxyliques, tels que des citrates, des phtalates, des adipates, des carbonates, de tartrates, des phosphates, des sébaçates; les esters issus de la réaction d'un acide monocarboxylique de formule R₁₁COOH avec un diol de formule HOR₁₂OH avec R₁₁ et R₁₂, identiques ou différents, représentent une chaîne hydrocarbonée, comprenant de préférence de 3 à 15 atomes de carbone, linéaire, ramifiée ou cyclique, saturée ou insaturée comprenant éventuellement un ou plusieurs hétéroatomes tels que N, O, S;
- des dérivés oxyéthylénés, tels que les huiles oxyéthylénées, notamment les huiles végétales, telles que l'huile de ricin ;
- les dimethicone copolyols, notamment à groupements propyl polyoxypropylène;
- et leurs mélanges.

La composition peut également comprendre un agent épaississant qui peut en particulier être choisi parmi :
- les silices notamment hydrophobes, telles que celles décrites dans le document EP-A-898960, et par exemple commercialisées sous les références "AEROSIL R812^{®}" par la société Degussa, "CAB-O-SIL TS-530^{®}", "CAB-O-SIL TS-610^{®}", "CAB-O-SIL TS-720^{®}"par la société Cabot, "AEROSIL R972^{®}", "AEROSIL R974^{®}" par la société Degussa ;
- les argiles telles que la montmorillonite, les argiles modifiées telles que les bentones par exemple, l'hectorite stéaralkonium, la bentonite stéaralkonium,
- les alkyléther de polysaccharides (notamment dont le groupe alkyle comporte de 1 à 24 atomes de carbones, de préférence de 1 à 10, mieux de 1 à 6, et plus spécialement de 1 à 3) tels que ceux décrits dans le document EP-A-898958.

La quantité d'agent épaississant dans la composition selon l'invention peut aller de 0,01 à 15% en poids, par rapport au poids total de la composition, de préférence de 0,1 à 12% et mieux de 0,5 à 10% en poids.

La composition selon l'invention peut en outre comprendre une résine secondaire, en plus du polycondensat selon l'invention et du polymère filmogène, qui peut être choisie parmi les résines aryl-sulfonamide-formaldéhyde ou aryl-sulfonamide-époxy, les résines polyesters, les résines de type alkyde, les résines polyuréthannes, les résines polyester-polyuréthannes, les résines polyéther-polyuréthannes, les résines vinyliques et/ou acryliques, seules ou en mélange. Cette résine secondaire additionnelle peut être présente à raison de 1 à 20% en poids, de préférence de 2 à 15% en poids et mieux de 3 à 10% en poids par rapport au poids total de la composition.

La composition selon l'invention peut également comprendre au moins une cire d'origine végétale, animale, minérale ou de synthèse, voire siliconée. On peut en particulier citer, seules ou en mélange, les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de Montan; les ozokérites; les cires de polyéthylène; les cires obtenues par synthèse de Fischer-Tropsch; les huiles hydrogénées, les esters gras et les glycérides concrets à 25°C. On peut également utiliser des cires de silicone, parmi lesquelles on peut citer les alkyls, alcoxys et/ou esters de polyméthylsiloxane.
La quantité de cire dans la composition selon l'invention peut aller de 0,01 à 15% en poids, par rapport au poids total de la composition, de préférence de 0,1 à 10% et mieux de 0,5 à 15% en poids.

La composition selon l'invention peut également comprendre une ou plusieurs matières colorantes choisies parmi les composés pulvérulents comme les pigments, les charges, les nacres et les paillettes, et/ou les colorants liposolubles ou hydrosolubles,
Les matières colorantes, notamment pulvérulentes, peuvent être présentes, dans la composition, en une teneur de 0,01 à 50% en poids, par rapport au poids de la composition, de préférence de 0,1 à 40% en poids, voire de 1 à 30% en poids.
Par pigments, il faut comprendre des particules de toute forme, blanches ou colorées, minérales ou organiques, insolubles dans le milieu physiologique, destinées à colorer la composition.
Par nacres, il faut comprendre des particules de toute forme irisées, notamment produites par certains mollusques dans leur coquille ou bien synthétisées.
Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.
Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.
Les charges peuvent être minérales ou organiques, lamellaires ou sphériques. On peut citer le talc, le mica, la silice, le kaolin, les poudres de Nylon et de polyéthylène, de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.
Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter 0,01 à 20% du poids de la composition et mieux de 0,1 à 6 %.
Les colorants hydrosolubles sont par exemple le jus de betterave, le bleu de méthylène et peuvent représenter 0,01 à 6% du poids total de la composition.

La composition peut comprendre, en outre, d'autres ingrédients utilisés couramment dans les compositions cosmétiques. De tels ingrédients peuvent être choisis parmi les antioxydants, les parfums, les huiles essentielles, les conservateurs, les actifs cosmétiques, les hydratants, les vitamines, les céramides, les filtres solaires, les tensioactifs, les agents d'étalement, les agents mouillants, les agents dispersants, les anti-mousses, les neutralisants, les stabilisants, et leurs mélanges.
Bien entendu, l'homme du métier veillera à choisir ce ou ces éventuels composés complémentaires, et/ou leur quantité, de manière telle que les propriétés avantageuses de la composition pour l'utilisation selon l'invention ne soient pas, ou substantiellement pas, altérées par l'adjonction envisagée.

Les compositions selon l'invention peuvent se présenter sous toute forme acceptable et usuelle pour une composition cosmétique ou pharmaceutique.
Elles peuvent donc se présenter sous la forme d'une suspension, une dispersion notamment d'huile dans de l'eau grâce à des vésicules; une solution organique ou huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; d'une lotion, d'une crème, d'une pommade, d'une pâte souple, d'un onguent, d'un solide coulé ou moulé et notamment en stick ou en coupelle, ou encore de solide compacté.
De préférence, les compositions selon l'invention se présentent sous forme de solution organique.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres, des cils, des sourcils, des cheveux, du cuir cheveu ou des ongles; d'un produit solaire ou autobronzant; d'un produit capillaire.
Les compositions conformes à l'invention peuvent être utilisées pour le soin ou le maquillage des matières kératiniques telles que les cheveux, la peau, les cils, les sourcils, les ongles, les lèvres, le cuir chevelu et plus particulièrement les ongles.

En effet, les polymères selon l'invention trouvent une application particulièrement intéressante dans le domaine du maquillage des ongles. Les compositions de l'invention se présentent avantageusement sous forme de vernis à ongles, notamment comprenant les polycondensats selon l'invention employés comme résine secondaire, en combinaison avec une résine principale qui peut être choisie parmi les polymères filmogènes décrits ci-dessus.

Préférentiellement, les compositions cosmétiques, notamment de vernis à ongles, selon l'invention comprennent :
- 0,1 et 50% en poids, de préférence entre 2 et 35% en poids, notamment entre 5 et 20% en poids, et mieux entre 6 et 18% en poids, par rapport au poids de la composition cosmétique, de polycondensat selon l'invention, seul ou en mélange;
- 1 à 70% en poids, de préférence 2 à 60% en poids, et mieux de 5 à 45% en poids, par rapport au poids total de la composition cosmétique, de polymère filmogène, notamment choisi parmi les polymères cellulosiques tels que la nitrocellulose, l'acétate de cellulose, l'acétobutyrate de cellulose, l'acétopropionate de cellulose, l'éthyl cellulose; les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines aryl-sulfonamide époxy ou encore les résines éthyl tosylamide; les polymères d'origine naturelle; et leurs mélanges;
- 10 à 95% en poids, de préférence 15% à 80% en poids, et mieux 20 à 60% en poids, par rapport au poids total de la composition cosmétique, de solvant organique, notamment choisi parmi les cétones liquides à température ambiante; les alcools liquides à température ambiante; les éthers de propylène glycol liquides à température ambiante; les éthers cycliques; les esters à chaîne courte (ayant de 3 à 8 atomes de carbone au total); les éthers liquides à température ambiante; les alcanes liquides à température ambiante; les aldéhydes liquides à température ambiante; et leurs mélanges.
- éventuellement d'au moins une matière colorante, qui peut être présente, dans la composition, en une teneur de 0,01 à 50% en poids, par rapport au poids de la composition, de préférence de 0,1 à 40% en poids, voire de 1 à 30% en poids.

L'invention a encore pour objet un procédé de traitement cosmétique des matières kératiniques, notamment de la peau du corps ou du visage, des ongles, des cheveux et/ou des cils, comprenant l'application sur lesdites matières d'une composition cosmétique telle que définie précédemment.
Ce procédé selon l'invention permet notamment le maquillage des ongles, par application d'une composition de vernis à ongles selon l'invention.

L'invention est illustrée plus en détail dans les exemples suivants,

### Méthode de mesure de la viscosité

a/ La viscosité à 110°C du polymère est mesurée à l'aide d'un viscosimètre à cône plan de type BROOKFIELD CAP 1000+.
   Le cône-plan adapté est déterminé par l'homme du métier, sur la base de ses connaissances; notamment :
   - entre 50 et 500 mPa.s, on peut utiliser un cône 02
   - entre 500 et 1000 mPa.s : cône 03
   - entre 1000 et 4000 mPa.s : cône 05
   - entre 4000 et 10000 mPa.s : cône 06
b/ La viscosité à 25°C de la solution du polymère à 70% dans l'acétate de butyle est mesurée à l'aide d'un viscosimètre BROOKFIELD DV-I, à 30 tours/min en utilisant un mobile S62.

### Exemple 1 : Synthèse du pentaérythrityl benzoate/isophtalate/isostéarate

Dans un réacteur équipé d'une agitation mécanique, d'une arrivée d'argon et d'un système de distillation, on charge 227,5 g d'acide benzoïque, 72,8 g d'acide isostéarique et 118,3 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 18 heures. On refroidit à une température comprise entre 100 et 130°C puis on introduit 91 g d'acide isophtalique et on chauffe à nouveau progressivement jusqu'à 220°C pendant environ 11 heures.
On obtient ainsi 430 g de polycondensat pentaérythrityl benzoate/isophtalate/isostéarate sous la forme d'une huile épaisse qui se solidifie à température ambiante.

Le polycondensat présente les caractéristiques suivantes :
- Indice d'acide = 12,7
- Indice d'hydroxyle = 49
- η_{110°C} = 25,4 Poises (soit 2540 mPa.s)
- rapport entre le nombre de mole d'acide monocarboxylique aromatique et le nombre de mole d'acide monocarboxylique non aromatique : 7,28.

On prélève 420g de polycondensat obtenu ci-dessus, on le chauffe à 100-120°C et on coule lentement 180g d'acétate de butyle sous agitation puis on clarifie par filtration à chaud sur fritté n°2.
On obtient après refroidissement à température ambiante 600 g de solution de polycondensat à 70% dans l'acétate de butyle, se présentant sous la forme d'un liquide visqueux jaune pâle et possédant une viscosité à 25°C d'environ 800 centipoises (mPa.s)

### Exemple 2 : Synthèse du pentaérythrityl benzoate/isophtalate/laurate/PDMS

Dans un réacteur équipé d'une agitation mécanique, d'une arrivée d'argon et d'un système de distillation, on charge 150 g d'acide benzoïque, 165 g d'acide laurique et 110 g de pentaérythritol, puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 15 heures. On refroidit à une température comprise entre 100 et 130°C puis on introduit 90 g d'acide isophtalique et 50 g de Silicone α,ω diol X22-160AS de Shin-Etsu, et on chauffe à nouveau progressivement jusqu'à 220°C pendant environ 11 heures.
On obtient ainsi 510 g de polycondensat pentaérythrityl benzoate/isophtalate/laurate /PDMS sous la forme d'une huile épaisse qui se solidifie à température ambiante.

Le polycondensat présente les caractéristiques suivantes :
- Indice d'acide = 28,7
- Indice d'hydroxyle = 85
- η_{110°C} = 2,1 Poises (soit 210 mPa.s)
- rapport entre le nombre de mole d'acide monocarboxylique aromatique et le nombre de mole d'acide monocarboxylique non aromatique : 1,49.

On prélève 500 g de polycondensat obtenu ci-dessus, on le chauffe à 70°C et on coule lentement 215 g d'acétate d'éthyle sous agitation puis on clarifie par filtration à chaud sur fritté n°2. On obtient après refroidissement à température ambiante 705 g de solution de polycondensat à 70% dans l'acétate d'éthyle se présentant sous la forme d'un liquide visqueux jaune pâle et possédant une viscosité à 25°C d'environ 165 centipoises (mPa.s).

### Exemple 3 : Synthèse du pentaérythrityl benzoate/isophtalate/laurate

Dans un réacteur équipé d'une agitation mécanique, d'une arrivée d'argon et d'un système de distillation, on charge 165 g d'acide benzoïque, 160 g d'acide laurique et 120 g de pentaérythritol puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 15 heures. On refroidit à une température comprise entre 100 et 130°C puis on introduit 100 g d'acide isophtalique et on chauffe à nouveau progressivement jusqu'à 220°C pendant environ 12 heures.
On obtient ainsi 510 g de polycondensat pentaérythrityl benzoate/isophtalate/laurate sous la forme d'une huile épaisse qui se solidifie à température ambiante.

Le polycondensat présente les caractéristiques suivantes :
- Indice d'acide = 20,4
- Indice d'hydroxyle = 66
- η_{110°C} = 4,7 Poises (soit 470 mPa.s)
- rapport entre le nombre de mole d'acide monocarboxylique aromatique et le nombre de mole d'acide monocarboxylique non aromatique : 1,69.

On prélève 500 g de polycondensat obtenu ci-dessus, le chauffe à 70°C et on coule lentement 215 g d'acétate d'éthyle sous agitation puis on clarifie par filtration à chaud sur fritté n°2. On obtient après refroidissement à température ambiante 700 g de solution de polycondensat à 70% dans l'acétate d'éthyle se présentant sous la forme d'un liquide visqueux jaune pâle et possédant une viscosité à 25°C d'environ 310 centipoises (mPa.s).

### Exemple 4 : Synthèse du pentaérythrityl benzoate/phtalate/laurate

Dans un réacteur équipé d'une agitation mécanique, d'une arrivée d'argon et d'un système de distillation, on charge 185 g d'acide benzoïque, 174 g d'acide laurique et 114,6 g de pentaérythritol puis on chauffe progressivement, sous un léger courant d'argon, à 110-130°C pour obtenir une solution homogène. On augmente ensuite progressivement la température jusqu'à 180°C et on la maintient pendant environ 2 heures. On augmente de nouveau la température jusqu'à 220°C et on la maintient jusqu'à ce qu'on obtienne un indice d'acide inférieur ou égal à 1, ce qui prend environ 18 heures. On refroidit à une température comprise entre 100 et 130°C puis on introduit 80 g d'anhydride phtalique et on chauffe à nouveau progressivement jusqu'à 220°C pendant environ 8 heures. On ajoute 15 g de pentaérythritol et on maintient 8 heures à 220°C.
On obtient ainsi 512 g de polycondensat pentaérythrityl benzoate/phtalate/laurate sous la forme d'une huile épaisse qui se solidifie à température ambiante.

Le polycondensat présente les caractéristiques suivantes :
- Indice d'acide = 13,0
- Indice d'hydroxyle = 60
- η_{110°C} = 0,9 Poises (soit 90 mPa.s)
- rapport entre le nombre de mole d'acide monocarboxylique aromatique et le nombre de mole d'acide monocarboxylique non aromatique : 1,74.

### Exemple 5

On a préparé un vernis à ongles coloré de teinte rouge cerise ayant la composition suivante :
- Nitrocellulose à 30% d'isopropanol 14 g
- Résine alkyde Beckosol ODE-230-70E à 70% dans l'acétate d'éthyle 5,2 g
- solution à 70% dans l'acétate de butyle du polymère de l'exemple 1 10,8 g
- Isopropanol 3,6 g
- Acétate d'éthyle 23 g
- Acétate de butyle 33,5 g
- Acétylcitrate de tributyle 3,4 g
- N-éthyl o,p-toluènesulfonamide 3,4 g
- acide citrique 0,05 g
- Hectorite modifiée 1,3 g
- Pigments (laques) 1,3 g

Le vernis s'applique facilement et forme un film très brillant et très résistant aux agressions extérieures.

### Exemple 6

On a préparé un vernis à ongles coloré de teinte rouge cerise ayant la composition suivante :
- Nitrocellulose à 30% d'isopropanol 14 g
- Résine alkyde Beckosol ODE-230-70E à 70% dans l'acétate d'éthyle 5,2 g
- solution à 70% dans l'acétate d'éthyle du polymère de l'exemple 2 11,2 g
- Isopropanol 3,6 g
- Acétate d'éthyle 19,5 g
- Acétate de butyle 36,5 g
- Acétylcitrate de tributyle 3,4 g
- N-éthyl o,p-toluènesulfonamide 3,4 g
- acide citrique 0,05 g
- Hectorite modifiée 1,3 g
- Pigments (laques) 1,3 g

Le vernis s'applique facilement et forme un film très brillant et très résistant aux agressions extérieures.

### Exemple 7

On a préparé un vernis à ongles coloré ayant la composition suivante (% en poids):
- Nitrocellulose à 30% d'isopropanol 14 %
- Solution à 70% dans l'acétate de butyle du polymère de l'exemple 1 17 %
- Isopropanol 3,6 %
- Acétate d'éthyle 23 %
- Acétate de butyle qsp 100%
- Acétylcitrate de tributyle 3,2 %
- N-éthyl o,p-toluènesulfonamide 3,6 %
- acide citrique 0,05 %
- Hectorite modifiée 1,3%
- Pigments (laques) 1,3 %

Le vernis s'applique facilement et forme un film très brillant et très résistant aux agressions extérieures.

## Revendications

1. Composition cosmétique ou pharmaceutique comprenant, dans un milieu cosmétiquement ou pharmaceutiquement acceptable, au moins un polycondensat susceptible d'être obtenu par réaction :
- de 15 à 30% en poids, par rapport au poids total du polycondensat, d'au moins un polyol hydrocarboné saturé, linéaire ou ramifié, comprenant 3 à 18 atomes de carbone, et 3 à 6 groupes hydroxyles (OH);
- de 5 à 40% en poids, par rapport au poids total du polycondensat, d'au moins un acide monocarboxylique non aromatique de formule RCOOH, dans laquelle R est un radical hydrocarboné saturé, linéaire, ramifié et/ou cyclique, comprenant 5 à 31 atomes de carbone;
- de 10 à 55% en poids, par rapport au poids total du polycondensat, d'au moins un acide monocarboxylique aromatique de formule R'COOH, dans laquelle R' est un radical benzoïque ou naphtoïque, éventuellement substitué par 1 à 3 radicaux alkyles, saturés ou insaturés, linéaires, ramifiés et/ou cycliques, comprenant 1 à 32 atomes de carbone;
- de 10 à 25% en poids, par rapport au poids total du polycondensat, d'au moins un acide polycarboxylique comprenant 2 à 4 groupes carboxyliques COOH, choisi parmi les acides polycarboxyliques aliphatiques saturés, linéaires, comprenant 2 à 20 atomes de carbone; et les acides polycarboxyliques aromatiques comprenant 8 à 12 atomes de carbone;
et/ou un anhydride cyclique choisi parmi l'anhydride phtalique, l'anhydride trimellitique, l'anhydride maléïque et l'anhydride succinique.

2. Composition selon la revendication 1, dans laquelle le polyol comprend 3 à 4 groupes hydroxyles.

3. Composition selon l'une des revendication précédentes, dans laquelle le polyol est un composé hydrocarboné saturé, linéaire ou ramifié, comprenant 4 à 10 atomes de carbone et 3 à 6 groupes hydroxy (OH).

4. Composition selon l'une des revendications précédentes, dans laquelle le polyol est choisi parmi le glycérol, le pentaérythritol, le sorbitol et leurs mélanges; et encore mieux est du pentaérythritol.

5. Composition selon l'une des revendications précédentes, dans laquelle le polyol, ou le mélange de polyol, représente 16 à 28% en poids, et mieux 18 à 25% en poids, du poids total du polycondensat.

6. Composition selon l'une des revendications précédentes, dans laquelle l'acide monocarboxylique non aromatique est de formule RCOOH, dans laquelle R est un radical hydrocarboné saturé, linéaire, ramifié et/ou cyclique, comprenant 7 à 27 atomes de carbone, et encore mieux 9 à 19 atomes de carbone, voire 11 à 17 atomes de carbone.

7. Composition selon l'une des revendications précédentes, dans lesquelles le radical R est linéaire ou ramifié, et préférentiellement en C5-C31.

8. Composition selon l'une des revendications précédentes, dans laquelle l'acide monocarboxylique non aromatique est choisi parmi, seul ou en mélange, l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide décanoïque, l'acide isononanoïque, l'acide laurique, l'acide tridécanoïque, l'acide myristique, l'acide palmitique, l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentylpropionique, l'acide cyclohexanecarboxylique, l'acide cyclohexylacétique, l'acide 4-cyclohexylbutyrique.

9. Composition selon l'une des revendications précédentes, dans laquelle l'acide monocarboxylique non aromatique est choisi parmi l'acide 2-éthylhexanoïque, l'acide isooctanoïque, l'acide laurique, l'acide palmitique, l'acide isostéarique, et leurs mélanges, et encore mieux l'acide isostéarique seul.

10. Composition selon l'une des revendications précédentes, dans laquelle l'acide monocarboxylique non aromatique, ou le mélange desdits acides, représente 8 à 38% en poids, et mieux 10 à 35% en poids, du poids total du polycondensat.

11. Composition selon l'une des revendications précédentes, dans laquelle l'acide monocarboxylique aromatique est choisi parmi, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque.

12. Composition selon l'une des revendications précédentes, dans laquelle l'acide monocarboxylique aromatique est choisi parmi l'acide benzoïque, l'acide o-toluique, l'acide m-toluique, l'acide 1-naphtoïque, seuls ou en mélanges; et encore mieux l'acide benzoïque seul.

13. Composition selon l'une des revendications précédentes, dans laquelle l'acide monocarboxylique aromatique, ou le mélange desdits acides, représente 20 à 52% en poids, voire 22 à 52% en poids, et mieux 25 à 50% en poids, du poids total du polycondensat.

14. Composition selon l'une des revendications précédentes, dans laquelle l'acide polycarboxylique ou son anhydride est choisi parmi, seul ou en mélange :
- les acides dicarboxyliques tels que l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide pimélique, l'acide sébacique, l'acide azélaïque, l'acide glutarique, l'acide adipique, l'acide fumarique, l'acide maléïque;
- les acides tricarboxyliques tels que l'acide cyclohexanetricarboxylique, l'acide trimellitique, l'acide 1,2,3-benzènetricarboxylique, l'acide 1,3,5-benzènetricarboxylique;
- les acides tétracarboxyliques tels que l'acide butanetétracarboxylique et l'acide pyroméllitique,
- les anhydrides cycliques de ces acides et notamment l'anhydride phtalique, l'anhydride trimellitique, l'anhydride maléïque et l'anhydride succinique.

15. Composition selon l'une des revendications précédentes, dans laquelle l'acide polycarboxylique ou son anhydride est choisi parmi l'anhydride phtalique et/ou l'acide isophtalique, et mieux l'acide isophtalique seul.

16. Composition selon l'une des revendications précédentes, dans laquelle l'acide polycarboxylique et/ou son anhydride cyclique représente 11 à 22% en poids, et mieux 12 à 20% en poids, du poids total du polycondensat.

17. Composition selon l'une des revendications précédentes, dans laquelle le polycondensat comprend en outre au moins une silicone à fonction hydroxyle (OH) et/ou carboxylique (COOH).

18. Composition selon la revendication 17, dans laquelle la silicone a une masse moléculaire moyenne en poids (Mw) comprise entre 300 et 20000, notamment 400 et 10 000, voire 800 et.4000.

19. Composition selon l'une des revendications 17 à 18, dans laquelle la silicone est de formule : 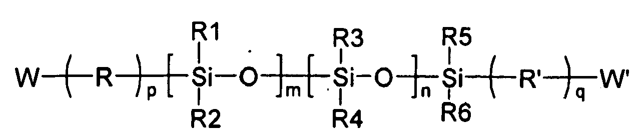 dans laquelle :
- W et W' sont, indépendamment l'un de l'autre, OH ou COOH; de préférence W=W';
- p et q sont, indépendamment l'un de l'autre, égaux à 0 ou 1,
- R et R' sont, indépendamment l'un de l'autre, un radical divalent carboné, notamment hydrocarboné, saturé ou insaturé, voire aromatique, linéaire, ramifié et/ou cyclique; comprenant 1 à 12 atomes de carbone, notamment 2 à 8 atomes de carbone, et comprenant éventuellement en outre 1 ou plusieurs hétéroatomes choisis parmi O, S et N, notamment O (éther);
notamment R et/ou R' peuvent être de formule -(CH₂)ₐ- avec a=1-12, et notamment méthylène, éthylène, propylène, phénylène;
ou bien de formule -[(CH₂)ₓO]_{z}- avec x = 1, 2 ou 3 et z = 1-10; en particulier x=2 ou 3 et z=1-4; et mieux x=3 et z=1.
- R1 à R6 sont, indépendamment l'un de l'autre, un radical carboné linéaire, ramifié et/ou cyclique, saturé ou insaturé voire aromatique; comprenant 1 à 20 atomes de carbone, notamment 2 à 12 atomes de carbone; de préférence, R1 à R6 sont saturés ou bien aromatiques, et peuvent notamment être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyles, en particulier le radical cyclohexyle, les radicaux aryles, notamment phényle et naphtyle, les radicaux arylalkyles, notamment benzyle et phényléthyle, ainsi que les radicaux tolyle et xylyle.
- m et n sont, indépendamment l'un de l'autre, des entiers compris entre 1 et 140, et sont tels que la masse moléculaire moyenne en poids (Mw) de la silicone est comprise entre 300 et 20 000, notamment entre 400 et 10 000, voire entre 800 et 4000.

20. Composition selon l'une des revendications 17 à 19, dans laquelle la silicone est choisie parmi, seule ou en mélange, les polyalkylsiloxanes α,ω-diol ou α,ω-dicarboxylique, et notamment les polydiméthysiloxanes α,ω-diol et les polydiméthylsiloxanes α,ω-dicarboxylique; les polyarylsiloxanes α,ω-diol ou α,ω-dicarboxylique et notamment les polyphénylsiloxanes α,ω-diol ou α,ω-dicarboxylique; les polyarylsiloxanes à fonctions silanol tels que le polyphénylsiloxane; les polyalkylsiloxanes à fonctions silanol tels que le polydiméthylsiloxane; les polyaryllalkylsiloxanes à fonctions silanol tels que le polyphényl/méthylsiloxane ou encore le polyphényl/propylsiloxane.

21. Composition selon l'une des revendications 17 à 20, dans laquelle la silicone est choisie parmi les polydiméthysiloxanes α,ω-diol de masse moléculaire moyenne en poids (Mw) comprise entre 400 et 10 000, voire entre 500 et 5000, et notamment entre 800 et 4000.

22. Composition selon l'une des revendications 17 à 21, dans laquelle la silicone représente 0,1 à 15% en poids, notamment 1 à 10% en poids, voire 2 à 8% en poids, du poids total du polycondensat.

23. Composition selon l'une des revendications précédentes, dans laquelle le rapport entre le nombre de mole d'acide monocarboxylique aromatique et le nombre de mole d'acide monocarboxylique non aromatique est compris entre 1,2 et 8, en particulier entre 1,3 et 7,8, voire entre 1,4 et 7,5 et encore mieux entre 1,9 et 7.

24. Composition selon l'une des revendications précédentes, dans laquelle le polycondensat est susceptible d'être obtenu par réaction :
- d'au moins un polyol choisi parmi, seul ou en mélange, le 1,2,6-hexanetriol, le triméthyloléthane, le triméthylolpropane, le glycérol; le pentaérythritol, l'érythritol, le diglycérol, le ditriméthylolpropane; le xylitol, le sorbitol, le mannitol, le dipentaérythritol et/ou le triglycérol;
présent de préférence en une quantité de 15 à 30% en poids, notamment 16 à 28% en poids, et mieux 18 à 25% en poids, par rapport au poids total du polycondensat final;
- d'au moins un acide monocarboxylique non aromatique choisi parmi, seul ou en mélange, l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide décanoïque, l'acide isononanoïque, l'acide laurique, l'acide tridécanoïque, l'acide myristique, l'acide palmitique, l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentylpropionique, l'acide cyclohexanecarboxylique, l'acide cyclohexylacétique, l'acide 4-cyclohexylbutyrique;
présent de préférence en une quantité de 5 à 40% en poids, notamment 8 à 38% en poids, et mieux 10 à 35% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque;
présent de préférence en une quantité de 10 à 55% en poids, notamment 20 à 52% en poids, et mieux 25 à 50% en poids, par rapport au poids total du polycondensat final; et
- d'au moins un acide polycarboxylique ou un de ses anhydrides, choisi parmi, seul ou en mélange, l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide pimélique, l'acide sébacique, l'acide azélaïque, l'acide glutarique, l'acide adipique, l'acide fumarique, l'acide maléïque; l'acide cyclohexanetricarboxylique, l'acide trimellitique, l'acide 1,2,3-benzènetricarboxylique, l'acide 1,3,5-benzènetricarboxylique; l'acide butanetétracarboxylique, l'acide pyroméllitique, l'anhydride phtalique, l'anhydride trimellitique, l'anhydride maléïque et l'anhydride succinique;
présent de préférence en une quantité de 10 à 25% en poids, notamment 11 à 22% en poids, et mieux 12 à 20% en poids, par rapport au poids total du polycondensat final.

25. Composition selon l'une des revendications précédentes, dans laquelle le polycondensat est susceptible d'être obtenu par réaction :
- d'au moins un polyol choisi parmi, seul ou en mélange, le glycérol, le pentaérythritol, le sorbitol et leurs mélanges, et encore mieux le pentaérythritol seul; présent en une quantité de 15 à 30% en poids, notamment 16 à 28% en poids, et mieux 18 à 25% en poids, par rapport au poids total du polycondensat final;
- d'au moins un acide monocarboxylique non aromatique choisi parmi, seul ou en mélange, l'acide 2-éthylhexanoïque, l'acide isooctanoïque, l'acide laurique, l'acide palmitique, l'acide isostéarique, et leurs mélanges, et encore mieux l'acide isostéarique seul; présent en une quantité de 5 à 40% en poids, notamment 8 à 38% en poids; et mieux 10 à 35% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide 1-naphtoïque, et encore mieux l'acide benzoïque seul; présent en une quantité de 10 à 55% en poids, notamment 20 à 52% en poids, et mieux 25 à 50% en poids, par rapport au poids total du polycondensat final; et
- d'au moins un acide polycarboxylique ou un de ses anhydrides, choisi parmi, seul ou en mélange, l'anhydride phtalique et l'acide isophtalique, et encore mieux l'acide isophtalique seul; présent en une quantité de 10 à 25% en poids, notamment 11 à 22% en poids, et mieux 12 à 20% en poids, par rapport au poids total du polycondensat final.

26. Composition selon l'une des revendications précédentes, dans laquelle le polycondensat présente au moins l'une des caractéristiques suivantes :
- un indice d'acide, exprimé en mg d'hydroxyde de potassium par g de polycondensat, supérieur ou égal à 8; notamment compris entre 8 et 40, et encore mieux compris entre 10 et 30; et/ou
- un indice d'hydroxyle exprimé en mg d'hydroxyde de potassium par g de polycondensat, supérieur ou égal à 30; notamment compris entre 30 et 100, et encore mieux compris entre 40 et 90.
- une viscosité, mesurée à 110°C, comprise entre 75 et 6000 mPa.s, notamment entre 80 et 5500 mPa.s, voire entre 90 et 5000 mPa.s, et encore mieux entre 200 et 4800 mPa.s;
- une solubilité dans l'acétate de butyle ou l'acétate d'éthyle, à raison d'au moins 50% en poids, à 25°C;
- une viscosité d'une solution du polymère dans l'acétate de butyle ou l'acétate d'éthyle, à 25°C, à une concentration de 70% en poids, comprise entre 100 et 1500 mPa.s, notamment entre 120 et 900 mPa.s.

27. Composition selon l'une des revendications précédentes, dans laquelle le polycondensat est présent en une quantité comprise entre 0,1 et 70% en poids, de préférence entre 2 et 50% en poids, notamment entre 3 et 35% en poids, voire entre 5 et 20% en poids, et mieux entre 6 et 18% en poids, par rapport au poids de la composition cosmétique ou pharmaceutique finale.

28. Composition selon l'une des revendications précédentes, dans laquelle le milieu cosmétiquement ou pharmaceutiquement acceptable comprend au moins un composé choisi parmi l'eau, les alcools, les polyols, les cétones, les esters, les éthers, les alcanes, les aldéhydes, les huiles carbonées, les huiles de silicone, les huiles de silicone fluorées, et leurs mélanges.

29. Composition selon l'une des revendications précédentes, dans laquelle le milieu cosmétiquement ou pharmaceutiquement acceptable comprend au moins un solvant organique choisi parmi :
- les cétones liquides à température ambiante (25°C) tels que la méthyléthylcétone, la méthylisobutylcétone, la diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les éthers de propylène glycol liquides à température ambiante tels que le monométhyléther de propylène glycol, l'acétate de monométhyléther de propylène glycol, le mono n-butyl éther de dipropylène glycol ;
- les éthers cycliques tels que la gamma-butyrolactone ;
- les esters à chaîne courte (ayant de 3 à 8 atomes de carbone au total) tels que l'acétate d'éthyle, l'acétate de méthyle, l'acétate de propyle, l'acétate d'isopropyle, l'acétate de n-butyle, l'acétate d'isopentyle, l'acétate de méthoxypropyle, le lactate de butyle ;
- les éthers liquides à température ambiante tels que le diéthyléther, le diméthyléther ou le dichlorodiéthyléther ;
- les alcanes liquides à température ambiante, notamment en C5-C12, tels que le décane, l'heptane, le dodécane, l'isododécane, le cyclohexane ;
- les aldéhydes liquides à température ambiante tels que le benzaldéhyde, l'acétaldéhyde ;
- et leurs mélanges.

30. Composition selon l'une des revendications précédentes, dans laquelle le milieu cosmétiquement ou pharmaceutiquement acceptable comprend au moins un solvant choisi parmi les esters à chaîne courte ayant de 3 à 8 atomes de carbone, tels que l'acétate d'éthyle, l'acétate de méthyle, l'acétate de propyle, l'acétate d'isopropyle, l'acétate de n-butyle, l'acétate d'isopentyle, l'acétate de méthoxypropyle, le lactate de butyle; les alcools liquides à température ambiante tels que l'éthanol, l'isopropanol, le diacétone alcool, le 2-butoxyéthanol, le cyclohexanol; et leurs mélanges.

31. Composition selon l'une des revendications précédentes, dans laquelle le milieu cosmétiquement ou pharmaceutiquement acceptable comprend au moins un constituant choisi parmi les huiles d'origine minérale, animale, végétale ou synthétique, carbonées, hydrocarbonées, fluorées et/ou siliconées; les polymères filmogènes; les agents auxiliaires de filmification; les agents épaississants; les résines secondaires; les cires d'origine végétale, animale, minérale ou de synthèse, voire siliconée; les matières colorantes; les antioxydants, les parfums, les huiles essentielles, les conservateurs, les actifs cosmétiques, les hydratants, les vitamines, les céramides, les filtres solaires, les tensioactifs, les agents d'étalement, les agents mouillants, les agents dispersants, les anti-mousses, les neutralisants, les stabilisants, et leurs mélanges.

32. Composition selon l'une des revendications précédentes, comprenant :
- 0,1 et 50% en poids, de préférence entre 2 et 35% en poids, notamment entre 5 et 20% en poids, et mieux entre 6 et 18% en poids, par rapport au poids de la composition cosmétique, de polycondensat selon l'une des revendications précédentes, seul ou en mélange;
- 1 à 70% en poids, de préférence 2 à 60% en poids, et mieux de 5 à 45% en poids, par rapport au poids total de la composition cosmétique, de polymère filmogène, notamment choisi parmi les polymères cellulosiques tels que la nitrocellulose, l'acétate de cellulose, l'acétobutyrate de cellulose, l'acétopropionate de cellulose, l'éthyl cellulose; les polyuréthanes, les polymères acryliques, les polymères vinyliques, les polyvinylbutyrals, les résines alkydes, les résines issues des produits de condensation d'aldéhyde tels que les résines arylsulfonamide formaldéhyde comme la résine toluène sulfonamide formaldéhyde, les résines arylsulfonamide époxy ou encore les résines éthyl tosylamide; les polymères d'origine naturelle; et leurs mélanges;
- 10 à 95% en poids, de préférence 15% à 80% en poids, et mieux 20 à 60% en poids, par rapport au poids total de la composition cosmétique, de solvant organique, notamment choisi parmi les cétones liquides à température ambiante; les alcools liquides à température ambiante; les éthers de propylène glycol liquides à température ambiante; les éthers cycliques; les esters à chaîne courte (ayant de 3 à 8 atomes de carbone au total); les éthers liquides à température ambiante; les alcanes liquides à température ambiante; les aldéhydes liquides à température ambiante; et leurs mélanges.
- éventuellement d'au moins une matière colorante, qui peut être présente, dans la composition, en une teneur de 0,01 à 50% en poids, par rapport au poids de la composition, de préférence de 0,1 à 40% en poids, voire de 1 à 30% en poids.

33. Composition selon l'une des revendications précédentes, se présentant sous la forme d'un produit de soin et/ou de maquillage de la peau du corps ou du visage, des lèvres, des cils, des sourcils, des cheveux, du cuir cheveu ou des ongles; d'un produit solaire ou autobronzant; d'un produit capillaire.

34. Composition selon l'une des revendications précédentes, se présentant sous la forme d'un vernis à ongles.

35. Procédé de traitement cosmétique des matières kératiniques, notamment de la peau du corps ou du visage, des ongles, des cheveux et/ou des cils, comprenant l'application sur lesdites matières d'une composition cosmétique telle que définie à l'une des revendications 1 à 34.

36. Procédé cosmétique de maquillage des ongles, comprenant l'application sur lesdits ongles d'une composition cosmétique telle que définie à l'une des revendications 1 à 34.

37. Polycondensat susceptible d'être obtenu par réaction:
- d'au moins un polyol choisi parmi, seul ou en mélange, le 1,2,6-hexanetriol, le triméthyloléthane, le triméthylolpropane, le glycérol; le pentaérythritol, l'érythritol, le diglycérol, le ditriméthylolpropane; le xylitol, le sorbitol, le mannitol, le dipentaérythritol et/ou le triglycérol; présent en une quantité de 15 à 30% en poids, notamment 16 à 28% en poids, et mieux 18 à 25% en poids, par rapport au poids total du polycondensat final;
- d'au moins un acide monocarboxylique non aromatique choisi parmi, seul ou en mélange, l'acide caproïque, l'acide caprylique, l'acide isoheptanoïque, l'acide 4-éthylpentanoïque, l'acide 2-éthylhexanoïque, l'acide 4,5-diméthylhexanoïque, l'acide 2-heptylheptanoïque, l'acide 3,5,5-triméthylhexanoïque, l'acide octanoïque, l'acide isooctanoïque, l'acide nonanoïque, l'acide décanoïque, l'acide isononanoïque, l'acide laurique, l'acide tridécanoïque, l'acide myristique, l'acide palmitique, l'acide stéarique, l'acide isostéarique, l'acide arachidique, l'acide béhénique, l'acide cérotique (hexacosanoïque); l'acide cyclopentanecarboxylique, l'acide cyclopentaneacétique, l'acide 3-cyclopentylpropionique, l'acide cyclohexanecarboxylique, l'acide cyclohexylacétique, l'acide 4-cyclohexylbutyrique; présent en une quantité de 5 à 40% en poids, notamment 8 à 38% en poids, et mieux 10 à 35% en poids, par rapport au poids total du polycondensat final;
- d'au moins un acide monocarboxylique aromatique choisi parmi, seul ou en mélange, l'acide benzoïque, l'acide o-toluique, l'acide m-toluique, l'acide p-toluique, l'acide 1-naphtoïque, l'acide 2-naphtoïque, l'acide 4-tert-butyl benzoïque, l'acide 1-méthyl-2-naphtoïque, l'acide 2-isopropyl-1-naphtoïque; présent en une quantité de 10 à 55% en poids, notamment 20 à 52% en poids, et mieux 25 à 50% en poids, par rapport au poids total du polycondensat final; et
- d'au moins un acide polycarboxylique ou un de ses anhydrides, choisi parmi, seul ou en mélange, l'acide décanedioïque, l'acide dodécanedioïque, l'acide cyclopropanedicarboxylique, l'acide cyclohexanedicarboxylique, l'acide cyclobutanedicarboxylique, l'acide naphtalène-1,4-dicarboxylique, l'acide naphtalène-2,3-dicarboxylique, l'acide naphtalène-2,6-dicarboxylique, l'acide subérique, l'acide oxalique, l'acide malonique, l'acide succinique, l'acide phtalique, l'acide téréphtalique, l'acide isophtalique, l'acide pimélique, l'acide sébacique, l'acide azélaïque, l'acide glutarique, l'acide adipique, l'acide fumarique, l'acide maléïque; l'acide cyclohexanetricarboxylique, l'acide trimellitique, l'acide 1,2,3-benzènetricarboxylique, l'acide 1,3,5-benzènetricarboxylique; l'acide butanetétracarboxylique, l'acide pyroméllitique, l'anhydride phtalique, l'anhydride trimellitique, l'anhydride maléïque et l'anhydride succinique; présent en une quantité de 10 à 25% en poids, notamment 11 à 22% en poids, et mieux 12 à 20% en poids, par rapport au poids total du polycondensat final.

38. Polycondensat selon la revendication 37, dans lequel le polycondensat comprend en outre au moins une silicone à fonction hydroxyle (OH) et/ou carboxylique (COOH).

39. Polycondensat selon l'une des revendications 37 à 38, dans lequel la silicone est de formule : 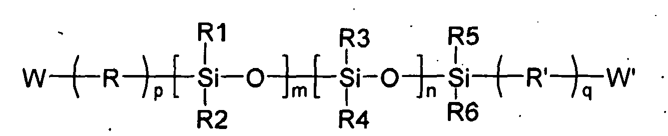 dans laquelle:
- W et W' sont, indépendamment l'un de l'autre, OH ou COOH; de préférence W=W';
- p et q sont, indépendamment l'un de l'autre, égaux à 0 ou 1,
- R et R' sont, indépendamment l'un de l'autre, un radical divalent carboné, notamment hydrocarboné, saturé ou insaturé, voire aromatique, linéaire, ramifié et/ou cyclique; comprenant 1 à 12 atomes de carbone, notamment 2 à 8 atomes de carbone, et comprenant éventuellement en outre 1 ou plusieurs hétéroatomes choisis parmi O, S et N, notamment O (éther);
notamment R et/ou R' peuvent être de formule -(CH₂)ₐ- avec a=1-12, et notamment méthylène, éthylène, propylène, phénylène;
ou bien de formule -[(CH₂)ₓ O]_{z}- avec x = 1, 2 ou 3 et z = 1-10; en particulier x=2 ou 3 et z=1-4; et mieux x=3 et z=1.
- R1 à R6 sont, indépendamment l'un de l'autre, un radical carboné linéaire, ramifié et/ou cyclique, saturé ou insaturé voire aromatique; comprenant 1 à 20 atomes de carbone, notamment 2 à 12 atomes de carbone; de préférence, R1 à R6 sont saturés ou bien aromatiques, et peuvent notamment être choisis parmi les radicaux alkyles, en particulier les radicaux méthyle, éthyle, propyle, isopropyle, butyle, pentyle, hexyle, octyle, décyle, dodécyle et octadécyle, les radicaux cycloalkyles, en particulier le radical cyclohexyle, les radicaux aryles, notamment phényle et naphtyle, les radicaux arylalkyles, notamment benzyle et phényléthyle, ainsi que les radicaux tolyle et xylyle.
- m et n sont, indépendamment l'un de l'autre, des entiers compris entre 1 et 140, et sont tels que la masse moléculaire moyenne en poids (Mw) de la silicone est comprise entre 300 et 20 000, notamment entre 400 et 10 000, voire entre 800 et 4000.

40. Polycondensat selon l'une des revendications 37 à 39, dans lequel la silicone est choisie parmi, seule ou en mélange, les polyalkylsiloxanes α,ω-diol ou α,ω-dicarboxylique, et notamment les polydiméthysiloxanes α,ω-diol et les polydiméthylsiloxanes α,ω-dicarboxylique; les polyarylsiloxanes α,ω-diol ou α,ω-dicarboxylique et notamment les polyphénylsiloxanes α,ω-diol ou α,ω-dicarboxylique; les polyarylsiloxanes à fonctions silanol tels que le polyphénylsiloxane; les polyalkylsiloxanes à fonctions silanol tels que le polydiméthylsiloxane; les polyaryl/alkylsiloxanes à fonctions silanol tels que le polyphényl/méthylsiloxane ou encore le polyphényl/propylsiloxane.

41. Polycondensat selon l'une des revendications 37 à 40, dans lequel la silicone représente 0,1 à 15% en poids, notamment 1 à 10% en poids, voire 2 à 8% en poids, du poids total du polycondensat.

42. Procédé de préparation des polycondensats selon l'une des revendications 37 à 41, consistant:
- à mélanger le polyol et les acides monocarboxyliques aromatiques et non aromatiques,
- à chauffer le mélange sous atmosphère inerte, d'abord jusqu'à la température de fusion (généralement 100-130°C) et ensuite à une température comprise entre 150 et 220°C jusqu'à consommation complète des acides monocarboxyliques, puis
- à éventuellement refroidir le mélange à une température comprise entre 90 et 150°C,
- à ajouter l'acide polycarboxylique et/ou l'anhydride cyclique, et optionnellement la silicone à fonctions hydroxyles ou carboxyliques, puis
- à chauffer à nouveau à une température inférieure ou égale à 220°C.

43. Procédé selon la revendication 42, dans lequel on ajoute un ou plusieurs agents antioxydants dans le milieu réactionnel, notamment à une concentration pondérale comprise entre 0,01 et 1%, par,rapport au poids total de monomères.

44. Procédé selon la revendication 43, dans lequel l'antioxydant est choisi parmi le BHT, le BHA, le TBHQ, le 1,3,5-trimethyl-2,4,6,tris(3,5-di-tertbutyl-4-hydroxybenzyl)-benzène, l'octadecyl-3,5,di-tertbutyl-4-hydroxycinnamate, le tetrakis-methylene-3-(3,5-di-tertbutyl-4-hydroxy-phenyl)propionate méthane, l'octadecyl-3-(3,5-di-tertbutyl-4-hydroxyphenyl)propionate 2,5-di-tertbutyl hydroquinone, le 2,2-methyl-bis-(4-methyl-6-tertbutyl phénol), le 2,2-methylene-bis-(4-ethyl-6-tertbutyl phénol), le 4,4-butylidene-bis(6-tertbutyl-m-cresol), le N,N-hexamethylene bis(3,5-di-tertbutyl-4-hydroxyhydrocinnamamide), le pentaerythritol tetrakis (3-(3,5-di-tertbutyl-4-hydroxyphenyl)propionate); l'octadecyl 3-(3,5-di-tertbutyl-4-hydroxphenyl) propionate; la 1,3,5-tris(3,5-di-tertbutyl-4-hydroxybenzyl)-1,3,5-triazine-2,4,6(1H,3H,5H)trione; le di(stearyl)pentaerythritol diphosphite, le tris(2,4-ditertbutyl phenyl)phosphite; le dilauryl thiodipropionate; le bis(2,4-di-tertbutyl)pentaerythritol diphosphite; le bis(2,4-bis)[2-phénylpropan-2-yl]phényl)pentaérythritol diphosphite, le triphénylphosphite, le (2,4-ditertbutylphenyl)pentaerythritol diphosphite; le tris(nonylphenyl)phosphite; le mélange 1:1 de N,N-hexamethylenebis(3,5-di-tertbutyl-4-hydroxy-hydrocinnamamide) et de tris(2,4-di-tertbutylphenyl)phosphate; le tétrakis (2,4-di-tert-butylphényl)phosphite; le distéarylthiodipropionate; le 2,4-bis(octylthiométhyl)o-crésol; le 4,6-bis(dodécylthiométhyl)o-crésol.

## Claims

1. Cosmetic or pharmaceutical composition comprising, in a cosmetically or pharmaceutically acceptable medium, at least one polycondensate that may be obtained by reacting:
- from 15% to 30% by weight, relative to the total weight of the polycondensate, of at least one linear or branched, saturated hydrocarbon-based polyol comprising 3 to 18 carbon atoms and 3 to 6 hydroxyl groups (OH);
- from 5% to 40% by weight, relative to the total weight of the polycondensate, of at least one non-aromatic monocarboxylic acid of formula RCOOH, in which R is a saturated, linear, branched and/or cyclic hydrocarbon-based radical comprising 5 to 31 carbon atoms;
- from 10% to 55% by weight, relative to the total weight of the polycondensate, of at least one aromatic monocarboxylic acid of formula R' COOH, in which R' is a benzoic or naphthoic radical, optionally substituted with 1 to 3 saturated or unsaturated, linear, branched and/or cyclic alkyl radicals comprising 1 to 32 carbon atoms;
- from 10% to 25% by weight, relative to the total weight of the polycondensate, of at least one polycarboxylic acid comprising 2 to 4 carboxylic groups COOH, chosen from saturated linear aliphatic polycarboxylic acids, comprising 2 to 20 carbon atoms; and aromatic polycarboxylic acids comprising 8 to 12 carbon atoms and/or a cyclic anhydride chosen from phthalic anhydride, trimellitic anhydride, maleic anhydride and succinic anhydride.

2. Composition according to Claim 1, in which the polyol comprises 3 to 4 hydroxyl groups.

3. Composition according to either of the preceding claims, in which the polyol is a saturated linear or branched hydrocarbon-based compound containing 4 to 10 carbon atoms, and 3 to 6 hydroxyl (OH) groups.

4. Composition according to one of the preceding claims, in which the polyol is chosen from glycerol, pentaerythritol and sorbitol, and mixtures thereof, and better still is pentaerythritol.

5. Composition according to one of the preceding claims, in which the polyol, or the polyol mixture, represents 16% to 28% by weight and better still 18% to 25% by weight relative to the total weight of the polycondensate.

6. Composition according to one of the preceding claims, in which the non-aromatic monocarboxylic acid is of formula RCOOH, in which R is a saturated, linear, branched and/or cyclic hydrocarbon-based radical containing 7 to 27 carbon atoms and better still 9 to 19 carbon atoms, or even 11 to 17 carbon atoms.

7. Composition according to one of the preceding claims, in which the radical R is linear or blanched, and is preferably of C5-C31.

8. Composition according to one of the preceding claims, is which the non-aromatic monocarboxylic acid is chosen, alone or as a mixture, from caproic acid, caprylic acid, isoheptanoic acid, 4-ethylpentanoic acid, 2-ethylhexanoic acid, 4,5-dimethylhexanoic acid, 2-heptylheptanoic acid, 3,5,5-trimethylhexanoic acid, octanoic acid, isooctanoic acid, nonanoic acid, decanoic acid, isononanoic acid, lauric acid, tridecanoic acid, myristic acid, palmitic acid, stearic acid, isostearic acid, arachidic acid, behenic acid, cerotic acid (hexacosanoic acid); cyclopentanecarboxylic acid, cyclopentaneacetic acid, 3-cyclopentylpropionic acid, cyclohexanecarboxylic acid, cyclohexylacetic acid or 4-cyclohexylbutyric acid.

9. Composition according to one of the preceding claims, in which the non-aromatic monocarboxylic acid is chosen from 2-ethylhexanoic acid, isooctanoic acid, lauric acid, palmitic acid and isostearic acid, and mixtures thereof, and better still isostearic acid alone.

10. Composition according to one of the preceding claims, in which the non-aromatic monocarboxylic acid or the mixture of the said acids, represents 3% to 38% and better still 10% to 35% by weight relative to the total weight of the polycondensat.

11. Composition according to one of the preceding claims, in which the aromatic monocarboxylic acid is chosen, alone or as a mixture, from benzoic acid, o-toluic acid, m-toluic acid, p-toluic acid, 1-naphthoic acid, 2-naphthoic acid, 4-tert-butylbenzoic acid, 1-methyl-2-naphthoic acid and 2-isopropyl-1-naphthoic acid.

12. Composition according to one of the preceding claims, in which the aromatic monocarboxylic acid is chosen from benzoic acid, o-toluic acid, m-toluic acid and 1-naphthoic acid, alone or as mixtures, and better still benzoic acid alone.

13. Composition according to one of the preceding claims, in which the aromatic monocarboxylic acid, or the mixture of the said acids, represents 20% to 52% by weight, or even 22% to 52% by weight and better still 25% to 50% by weight relative to the total weight of the polycondensate.

14. Composition according to one of the preceding claims, in which the polycarboxylic acid or the anhydride thereof is chosen, alone or as a mixture, from:
- dicarboxylic acids such as decanedioic acid, dodecanedioic acid, cyclopropanedicarboxylic acid, cyclohexanedicarboxylic acid, cyclobutanedicarboxylic acid, naphthalene-1,4-dicarboxylic acid, naphthalene-2,3-dicarboxylic acid, naphthalene-2, 6- dicarboxylic acid, suberic acid, oxalic acid, malonic acid, succinic acid, phthalic acid, terephthalic acid, isophthalic acid, pimelic acid, sebacic acid, azelaic acid, glutaric acid, adipic acid, fumaric acid or maleic acid;
- tricarboxylic acids such at cyclohexanetricarboxylic acid, trimellitic acid, 1,2,3-benzenetricarboxylic acid or 1,3,5-benzenetricarboxylic acid;
- tetracarboxylic acids such as butanetetracarboxylic acid and pyromellitic acid;
- cyclic anhydrides of these acids and especially phthalic anhydride, trimellitic anhydride, maleic anhydride and succinic anhydride.

15. Composition according to one of the preceding claims, in which the polycarboxylic acid or the anhydride thereof is chosen from phthalic anhydride and/or isophthalic acid, and better still isophthalic acid alone.

16. Composition according to one of the preceding claims, in which the polycarboxylic acid and/or the cyclic anhydrides thereof represents 11% to 22% by weight and better still 12% to 20% by weight relative to the total weight of the polycondensate.

17. Composition according to one of the preceding claims, in which the polycondensate also comprises at least one silicone containing hydroxyl (OH) and/or carboxylic (COOH) functions.

18. Composition according to Claim 17, in which the silicone has a weight-average molecular mass (Mw) of between 300 and 20 000, especially 400 and 10 000 or even 800 and 4000.

19. Composition according to either of Claims 17 and 18, in which the silicone is of the formula: 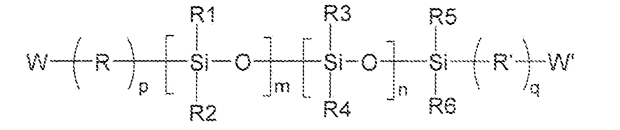 in which:
- W and W' are, independently of each other, OH or COOH; preferably, W = W';
- p and q are, independently of each other, equal to 0 or 1;
- R and R' are, independently of each other, a saturated or unsaturated, or even aromatic, linear, branched and/or cyclic carbon-based and especially hydrocarbon-based divalent radical; containing 1 to 12 carbon atoms and especially 2 to 8 carbon atoms, and optionally also comprising one or more heteroatoms chosen from O, S and N, especially O (ether);
R and/or R' may especially be of formula -(CH₂)ₐ- with a = 1-12, and especially methylene, ethylene, propylène or phenylene;
or alternatively of formula -[(CH₂)ₓO]_{z}- with x = 1, 2 or 3 and z = 1-10; in particular x = 2 or 3 and z = 1-4; and better still x = 3 and z = 1;
- R1 to R6 are, independently of each other, a linear, branched and/or cyclic, saturated or unsaturated, or even aromatic, carbon-based radical containing 1 to 20 carbon atoms and especially 2 to 12 carbon atoms; preferably, R1 to R6 are saturated or aromatic, and may be chosen especially from alkyl radicals, in particular methyl, ethyl, propyl, isopropyl, butyl, pentyl, hexyl, octyl, decyl, dodecyl and octadecyl radicals, cycloalkyl radicals, in particular the cyclohexyl radical, aryl radicals, especially phenyl and naphthyl, arylalkyl radicals, especially benzyl and phenylethyl, and also tolyl and xylyl radicals;
- m and n are, independently of each other, integers between 1 and 140, and are such that the weight-average molecular mass (Mw) of the silicone is between 300 and 20 000, especially between 400 and 10 000 or even between 800 and 4000.

20. Composition according to one of Claims 17 to 19, in which the silicone is chosen, alone or as a mixture, from α,ω-diol or α,ω-dicarboxylic polyalkylsiloxanes and especially α,ω-diol polydimethylsiloxanes and α,ω-dicarboxylic polydimethylsiloxanes; α,ω-diol or α,ω-dicarboxylic polyarylsiloxanes and especially α,ω-diol or α,ω-dicarboxylic polyphenylsiloxanes; polyarylsiloxanes containing silanol functions such as polyphenylsiloxane; polyalkylsiloxanes containing silanol functions such as polydimethylsiloxane; polyaryl/alkylsiloxanes containing silanol functions such as polyphenyl/methylsiloxane or polyphenyl/propylsiloxane.

21. Composition according to one of Claims 17 to 20, in which the silicone is chosen from α,ω-diol polydimethylsiloxanes with a weight-average molecular mass (Mw) of between 400 and 10 000, or even between 500 and 5000 and especially between 800 and 4000.

22. Composition according to one of Claims 17 to 21, in which the silicone represents 0.1% to 15% by weight, especially 1% to 10% by weight or even 2% to 8% by weight relative to the total weight of the polycondensate.

23. Composition according to one of the preceding claims, in which the ratio between the number of moles of aromatic monocarboxylic acid and the number of moles of non-aromatic monocarboxylic acid is between 1.2 and 8, in particular between 1.3 and 7.8, or even between 1.4 and 7.5 and better still between 1.9 and 7.

24. Composition according to one of the preceding claims, in which the polycondensate may be obtained by reacting ;
- at least one polyol chosen, alone or as a mixture, from 1,2,6-hexanetriol, trimethylolethane, trimethylolpropane, glycerol; pentaerythritol, erythritol, diglycerol, ditrimethylolpropane; xylitol, sorbitol, mannitol, dipentaerythritol and/or triglycerol ; preferably present in an amount of 15% to 30% by weight, especially 16% to 28% by weight and better still 18% to 25% by weight, relative to the total weight of the final polycondensate;
- at least one non-aromatic monocarboxylic acid chosen, alone or as a mixture; from caproic acid, caprylic acid, isoheptanoic acid, 4-ethylpentanoic acid, 2-ethylhexanoic acid, 4,5-dimethylhexanoic acid, 2-heptylheptanoic acid, 3,5,5-trimethylhexanoic acid, octanoic acid, isooctanoic acid, nonanoic acid, decanoic acid, isononanoic acid, lauric acid, tridecanoic acid, myristic acid, palmitic acid, stearic acid, isostearic acid, arachidic acid, behenic acid, cerotic acid (hexacosanoic acid), cyclopentanecarboxylic acid, cyclopentaneacetic acid, 3-cyclo-pentylpropionic acid, cyclohexanecarboxylic acid, cyclohexylacetic acid or 4 -cyclohexylbutyric acid; preferably present in an amount of 5% to 40% by weight, especially 8% to 38% by weight and better still 10% to 35% by weight relative to the total weight of the final polycondensate,
- at least one aromatic monocarboxylic acid chosen, alone or as a mixture, from benzoic acid, o-toluic acid, m-toluic acid, p-toluic acid, 1-naphthoic acid, 2-naphthoic acid, 4-tert-butylbenzoic acid, 1-methyl-2-naphthoic acid and 2-isopropyl-1-naphthoic acid; preferably present in an amount of 10% to 55% by weight, especially 20% to 52% by weight and better still 25% to 50% by weight relative to the total weight of the final polycondensate; and
- at least one polycarboxylic acid or an anhydride thereof, chosen, alone or as a mixture, from decanedioic acid, dodecanedioic acid, cyclopropanedicarboxylic acid, cyclohexanedicarboxylic acid, cyclobutanedicarboxylic acid, naphthalene-1,4-dicarboxylic acid, naphthalene-2,3-dicarboxylic acid, naphthalene-2,6-dicarboxylic acid, suberic acid, oxalic acid, malonic acid, succinic acid, phthalic acid, terephthalic acid, isophthalic acid, pimelic acid, sebacic acid, azelaic acid, glutaric acid, adipic acid, fumaric acid or maleic acid; cyclohexanetricarboxylic acid, trimellitic acid, 1,2,3 -benzenetricarboxylic acid, 1,3, 5-benzenetricarboxylic acid; butanetetracarboxylic acid, pyromellitic acid, phthalic anhydride, trimellitic anhydride, maleic anhydride and succinic anhydride;
preferably present in an amount of 10% to 25% by weight, especially 11% to 22% by weight and better still 12% to 20% by weight relative to the total weight of the final polycondensate.

25. Composition according to one of the preceding claims, in which the polycondensate may be obtained by reacting:
- at least one polyol chosen, alone or as a mixture, from glycerol, pentaerythritol and sorbitol, and mixtures thereon, and better still pentaerythritol alone; present in an amount of 15% to 30% by weight, especially 16% to 28% by weight and better still 18% to 25% by weight relative to the total weight of the final polycondensate;
- at least one non-aromatic monocarboxylic acid chosen, alone or as a mixture, from 2-ethylhexanoic acid, isooctanoic acid, lauric acid, palmitic acid and isostearic acid, and mixtures thereof, and better still isostearic acid alone; present in an amount of 5% to 40% by weight, especially 8% to 38% by weight and better still 10% to 35% by weight relative to the total weight of the final polycondensate;
- at least one aromatic monocarboxylic acid chosen, alone or as a mixture, from benzoic acid, o-toluic acid, m-toluic acid and 1-naphthoic acid, and better still benzoic acid alone; present in an amount of 10% to 55% by weight, especially 20% to 52% by weight and better still 25% to 50% by weight relative to the total weight of the final polycondensate; and
- at least one polycarboxylic acid or an anhydride thereof, chosen, alone or as a mixture, from phthalic anhydride and isophthalic acid, and better still isophthalic acid alone; present in an amount of 10% to 25% by weight, especially 11% to 22% by weight and better still 12% to 20% by weight relative to the total weight of the final polycondensate.

26. Composition according to one of the preceding claims, in which the polycondensate has at least one of the following characteristics:
an acid number, expressed in mg of potassium hydroxide per g of polycondensate, of greater than or equal to 8; especially between 8 and 40 and better still between 10 and 30; and/or
- a hydroxyl number, expressed in mg of potassium hydroxide per g of polycondensate, of greater than or equal to 30; especially between 30 and 100 and better still between 40 and 90,
- a viscosity, measured at 110°C, of between 75 and 6000 mPa.s, especially between 80 and 5500 mPa.s, or even between 90 and 5000 mPa.s and better still between 200 and 4800 mPa.s;
- a solubility in butyl acetate or ethyl acetate, in a proportion of at least 50% by weight, at 25°C;
- a viscosity of a solution of the polymer in butyl acetate or ethyl acetate, at 2.5°C, at a concentration of 70% by weight, of between 100 and 1500 mPa.s and especially between 120 and 900 mPa.s.

27. Composition according to one of the preceding claims, in which the polycondensate is present in an amount of between 0.1% and 70% by weight, preferably between 2% and 50% by weight, especially between 3% and 35% by weight, or even between 5% and 20% by weight, and better still between 6% and 18% by weight, relative to the weight of the final cosmetic or pharmaceutical composition.

28. Composition according to one of the preceding claims, 1 in which the cosmetically or pharmaceutically acceptable medium comprises at least one compound chosen from water, alcohols, polyols, ketones, esters, ethers, alkanes, aldehydes, carbon-based oils, silicone oils and fluorosilicone oils, and mixtures thereof.

29. Composition according to one of the preceding claims, in which the cosmetically or pharmaceutically acceptable medium comprises at least one organic solvent chosen from:
- ketones that are liquid at room temperature (25°C), such as methyl ethyl ketone, methyl isobutyl ketone, diisobutyl ketone, isophorone, cyclohexanone or acetone;
- alcohols that are liquid at room temperature, such as ethanol, isopropanol, diacetone alcohol, 2-butoxyethanol or cyclohexanol;
- propylene glycol ethers that are liquid at room temperature, such as propylene glycol monomethyl ether, propylene glycol monomethyl ether acetate or dipropylene glycol mono-n-butyl ether;
- cyclic ethers such as γ-butyrolactone;
- short-chain esters (containing from 3 to 8 carbon atoms in total) such as ethyl acetate, methyl acetate, propyl acetate, isopropyl acetate, n-butyl acetate, isopentyl acetate, methoxypropyl acetate or butyl lactate;
- ethers that are liquid at room temperature, such a diethyl ether, dimethyl ether or dichlorodiethyl ether;
- alkanes, especially of C5 -C12, that are liquid at room temperature, such as decane, heptane, dodecane, isododecane or cyclohexane;
- aldehydes that are liquid at room temperature, such as benzaldehyde or acetaldehyde;
- and mixtures thereof.

30. Composition according to one or the preceding claims, in which the cosmetically or pharmaceutically acceptable medium comprises at least one solvent chosen from short-chain esters containing from 3 to 8 carbon atoms, such as ethyl acetate, methyl acetate, propyl acetate, isopropyl acetate, n-butyl acetate, isopentyl acetate, methoxypropyl acetate, butyl lactate; alcohols that are liquid at room temperature, such as ethanol, isopropanol, diacetone alcohol, 2-butoxyethanol or cyclohexanol; and mixtures thereof.

31. Composition according to one of the preceding claims, or which the cosmetically or pharmaceutically acceptable medium comprises at least one constituent chosen from carbon-based, hydrocarbon-based, fluoro and/or silicone oils on mineral, animal, plant or synthetic origin; film-forming polymers; auxiliary film-forming agents; thickeners; secondary resins ; waxes of plant, animal, mineral or synthetic origin, or even silicone waxes; dyestuffs; antioxidants, fragrances, essential oils, preserving agents, cosmetic active agents, moisturizers, vitamins, ceramides, sunscreens, surfactants, spreading agents, wetting agents, dispersants, antifoams, neutralizers and stabilisers, and mixtures thereof.

32. Composition according to one of the preceding claims, comprising:
- 0.1% to 50% by weight, preferably between 2% and 35% by weight, especially between 5% and 20% by weight and better still between 6% and 18% by weight, relative to the weight of the cosmetic composition, of polycondensate according to one of the preceding claims, alone or as a mixture;
- 1% to 70% by weight, preferably 2% to 60% by weight and better still 5% to 45% by weight, relative to the total weight of the cosmetic composition, of film-forming polymer, chosen especially from cellulose-based polymers such as nitrocellulose, cellulose acetate, cellulose acetobutyrate, cellulose acetopropionate or ethylcellulose; polyurethanes, acrylic polymers; vinyl polymers, poly-vinyl butyrals, alkyd resins, resins derived from the products of condensation of aldehyde, such as arylsulfonamide-formaldehyde resins, for instance toluene sulfonamide-formaldehyde resin, arylsulfonamide-epoxy resins or ethyltosylamide resins; polymers of natural origin; and mixtures thereof;
- 10% to 95% by weight, preferably 15% to 80% by weight and better still 20% to 60% by weight, relative to the total weight of the cosmetic composition, of organic solvent, chosen especially from ketones that are liquid at room temperature; alcohols that are liquid at room temperature; propylene glycol ethers that are liquid at room temperature; cyclic ethers; short-chain esters (containing from 3 to 8 carbon atoms in total); ethers that are liquid at room temperature; alkanes that are liquid at room temperature; aldehydes that are liquid at room temperature; and mixtures thereof;
- optionally at least one dyestuff, which may be present in the composition in a content of from 0.01% to 50% by weight, preferably from 0.1% to 40% by weight or even from 1% to 30% by weight relative to the weight of the composition.

33. Composition according to one of the preceding claims, which is in the form of a care and/or makeup product for bodily or facial skin, the lips, the eyelashes, the eyebrows, the hair, the scalp or the nails; an antisun or self-tanning product; a haircare product.

34. Composition according to one of the preceding claims, which is in the form of a nail varnish.

35. Cosmetic process for treating keratin materials, especially bodily or facial skin, the nails, the hair and/or the eyelashes, comprising the application to the said materials on a cosmetic composition as defined in one of Claims 1 to 34.

36. Cosmetic process for making up the nails, comprising the application to the said nails of a cosmetic composition as defined in one of Claims 1 to 34.

37. Polycondensate that may be obtained by reacting:
- at least one polyol chosen, alone or as a mixture, from 1,2,6-hexanetriol, trimethylolethane, trimethylolpropane, glycerol; pentaerythritol, erythritol, diglycerol, ditrimethylolpropane; xylitol, sorbitol, mannitol, dipentaerythritol and/or triglycerol; present in an amount of 15% to 30% by weight, especially 16% to 28% by weight and better still 1.8% to 25% by weight, relative to the total weight or the final polycondensate;
- at least one non-aromatic monocarboxylic acid chosen, alone or as a mixture, from caproic acid, caprylic acid, isoheptanoic acid, 4-ethylpentanoic acid, 2-ethylhexanoic acid, 4,5-dimethylhexanoic acid, 2-heptylheptanoic acid, 3,5,5-trimethylhexanoic acid, octanoic acid, isooctanoic acid, nonanoic acid, decanoic acid, isononanoic acid, lauric acid, tridecanoic acid, myristic acid, palmitic acid, stearic acid, isostearic acid, arachidic acid, behenic acid, cerotic acid (hexacosanoic acid); cyclopentanecarboxylic acid, cyclopentaneacetic acid, 3-cyclopentylpropionic acid, cyclohexanecarboxylic acid, cyclohexylacetic acid or 4-cyclohexylbutyric acid; present in an amount of 5% to 40% by weight, especially 8% to 38% by weight and better still 10% to 35% by weight relative to the total weight of the final polycondensate;
- at least one aromatic monocarboxylic acid chosen, alone or as a mixture, from benzoic acid, o-toluic acid, m-toluic acid, p-toluic acid, 1-naphthoic acid, 2-naphthoic acid, 4-tert-butylbenzoic acid, 1-methyl-2-naphthoic acid and 2 - isopropyl-1-naphthoic acid: present in an amount of 10% to 55% by weight, especially 20% to 52% by weight and better still 25% to 50% by weight relative two the total weight of the final polycondensate; and
- at least one polycarboxylic acid or an anhydride thereof, chosen, alone or as a mixture, from decanedioic acid, dodecanedioic acid, cyclopropanedicarboxylic acid, cyclohexanedicarboxylic acid, cyclobutanedicarboxylic acid, naphthalene-1,4-dicarboxylic acid, naphthalene-2,3-dicarboxylic acid, naphthalene-2,6-dicarboxylic acid, suberic acid, oxalic acid, malonic acid, succinic acid, phthalic acid, terephthalic acid, isophthalic acid, pimelic acid, sebacic acid, azelaic acid, glutaric acid, adipic acid, fumaric acid or maleic acid; cyclohexanetricarboxylic acid, trimellitic acid, 1,2,3-benzenetricarboxylic acid, 1,3, 5-benzenetricarboxylic acid; butanetetracarboxylic acid, pyromellitic acid, phthalic anhydride, trimellitic anhydride, maleic anhydride and succinic anhydride; present in an amount of 10% to 25% by weight, especially 11% to 22% by weight and better still 12% to 20% by weight relative to the total weight of the final polycondensate.

38. Polycondensate according to Claim 37, in which the polycondensate also comprises at least one silicone containing hydroxyl (OH) and/or carboxylic (COOH) functions.

39. Polycondensate according to either of Claims 37 and 38, in which the silicone is of the formula; 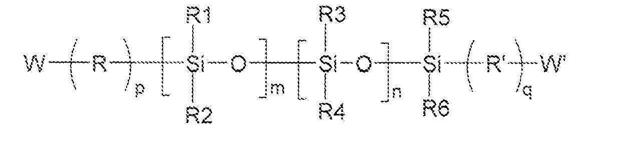 in which:
- W and W' are, independently of each other, OH or COOH; preferably, W = W';
- p and q are, independently of each other equal to 0 or 1;
- R and R' are, independently of each other, a saturated or unsaturated, or even aromatic, linear, branched and/or cyclic carbon-based and especially hydrocarbon-based divalent radical; containing 1 to 12 carbon atoms and especially 2 to 8 carbon atoms, and optionally also comprising one or more heteroatoms chosen from O, S and N, especially O (ether);
R and/or R' may especially be of formula -(CH₂)ₐ- with a = 1-12, and especially methylene, ethylene, propylène or phenylene;
or alternatively of formula -[(CH₂)ₓO]_{z}- with x **=** 1, 2 or 3 and z = 1-10; in particular x = 2 or 3 and
z = 1-4; and better still x = 3 and z = 1;
- R1 to R6 are, independently of each other, a linear, branched and/or cyclic, saturated or unsaturated, or even aromatic, carbon-based radical containing 1 to 20 carbon atoms and especially 2 to 12 carbon atoms;
preferably, R1 to R6 are saturated or aromatic, and may be chosen especially from alkyl radicals, in particular methyl, ethyl, propyl, isopropyl, butyl, pentyl, hexyl, octyl, decyl, dodecyl and octadecyl radicals, cycloalkyl radicals, in particular the cyclohexyl radical,aryl radicals, especially phenyl and naphthyl, arylalkyl radical, especially benzyl and phenylethyl, and also tolyl and xylyl radicals;
- m and n are, independently of each other, integers between 1 and 140, and are such that the weight-average molecular mass (Mw) of the silicons is between 300 and 20 000, especially between 400 and 10 000 or even between 800 and 4000.

40. Polycondensate according to one of Claims 37 to 39, in which the silicone is chosen, alone or as a mixture, from α,ω-diol or α,ω-dicarboxylic polyalkylsiloxanes and especially α,ω-diol polydimethylsiloxanes and α,ω-dicarboxylic polydimethylsiloxanes; α,ω-diol or α,ω-dicarboxylic polyarylsiloxanes and especially α,ω-diol or α,ω-dicarboxylic polyphenylsiloxanes; polyarylsiloxanes containing silanol functions such as polyphenylsiloxane; polyalkylsiloxanes containing silanol functions such as polydimethylsiloxane; polyaryl/alkylsiloxanes containing silanol functions such as polyphenyl/methylsiloxane or polyphenyl/propylsiloxane.

41. Polycondensate according to one of Claims 37 to 40, in which the silicone represents 0.1% to 15% by weight, especially 1% to 10% by weight or even 2% to 8% by weight relative to the total weight on the polycondensate.

42. Process for preparing polycondensates according to one of Claims 37 to 41, which consists in:
- mixing the polyol and the aromatic and non-aromatic monocarboxylic acids,
- heating the mixture under an inert atmosphere, first to the melting point (generally 100-130°C), and then to a temperature of between 150 and 220°C until the monocarboxylic acids have been totally consumed, then
- optionally cooling the mixture to a temperature of between 90 and 1.50°C,
- adding the polycarboxylic acid and/or the cyclic anhydride, and optionally the silicone containing hydroxyl or carboxylic functions, and then
- heating again to a temperature of less than or equal to 220°C.

43. Process according to Claim 42, in which one or more antioxidants are added to the reaction medium, especially in a weight concentration of between 0.01% and 1% relative to the total weight of monomers.

44. Process according to Claim 43, in which the antioxidant is chosen from BHT, BHA, TBHQ, 1,3,5-trimethyl-2,4,6-tris(3,5-di-tert-butyl-4-hydroxybenzyl)-benzene, octadecyl 3,5-di-tert-butyl-4-hydroxy-cinnamate, methanetetrakis[methylene-3-(3,5-di-tert-butyl-4-hydroxyphenyl) propionate], octadecyl 3-(3,5-di-tert-butyl-4-hydroxyphenyl)propionate 2,5-di-tert-butylhydroquinone, 2,2-methylbis(4-methyl-6-tert-butyl-phenol), 2,2-methylenebis(4-ethyl-6-tert-butylphenol), 4,4-butylidenebis(6-tert-butyl-m-cresol), N,N-hexa-methylenebis (3,5-di-tert-butyl-4-hydroxyhydrocinnamamide), pentaerythritoltetrakis (3-(3,5-di-tert-butyl-4-hydroxyphenyl)propionate); octadecyl 3-(3,5-di-tert-butyl-4-hydroxyphenyl)propionate, 1,3,5-tris(3,5-di-tert-butyl-4-hydroxybenzyl)-1,3,5-triazine-2,4,6-(1H,3H,5H)-trione; di(stearyl)pentaerythritol diphosphite, tris(2,4-di-tert-butylphenyl) phosphite; dilauryl thiodipropionate; bis(2,4-di-tert-butyl)pentaerythritol diphosphite; bis(2,4-bis)[2-phenylpropan-2-yl]phenyl)pentaerythritol diphosphite, triphenyl phosphite, (2,4-di-tert-butylphenyl)pentaerythritol diphosphite; tris(nonylphenyl) phosphite; the 1:1 mixture of N,N-hexamethylenebis(3,5-di-tert-butyl-4-hydroxyhydrocinnamamide) and of tris(2,4-di-tert-butyl-phenyl) phosphate; tetrakis(2,4-di-tert-butylphenyl) phosphite; distearyl thiodipropionate; 2,4-bis(octyl-thiomethyl)-o-cresol; 4,6-bis(dodecylthiomethyl)-o-cresol.

## Patentansprüche

1. Kosmetische oder pharmazeutische Zusammensetzung, die in einem kosmetisch oder pharmazeutisch akzeptablen Medium mindestens ein Polykondensat enthält, das erhältlich ist durch die Reaktion folgender Verbindungen:
- 15 bis 30 Gew.-% mindestens eines linearen oder verzweigten, gesättigten Polyols auf Kohlenwasserstoffbasis, das 3 bis 18 Kohlenstoffatome und 3 bis 6 Hydroxygruppen (OH) aufweist, bezogen auf das Gesamtgewicht, des Polykondensats;
- 5 bis 40 Gew.-% mindestens einer nichtaromatischen Monocarbonsäure der Formel RCOOH, wobei R eine lineare, verzweigte und/oder cyclische, gesättigte Kohlenwasserstoffgruppe mit 5 bis 31 Kohlenstoffatomen ist, bezogen auf das Gesamtgewicht des Polykondensats;
- 10 bis 55 Gew.-% mindestens einer aromatischen Monocarbonsäure der Formel R'COOH, wobei R' eine Benzoe-oder Naphthoegruppe ist, die gegebenenfalls mit 1 bis 3 gesättigten oder ungesättigten, linearen, verzweigten und/oder cyclischen Alkylgruppen mit 1 bis 32 Kohlenstoffatomen substituiert ist, bezogen auf das Gesamtgewicht des Polykondensats;
- 10 bis 25 Gew.-% mindestens einer Polycarbonsäure, die 2 bis 4 Carboxygruppen COOH die unter den gesättigten, linearen aliphatischen Polycarbonsäuren mit 2 bis 20 Kohlenstoffatomen und den aromatischen Polycarbonsäuren mit 8 bis 12 Kohlenstoffatomen ausgewählt ist; und/oder
eines cyclischen Anhydrids, das unter Phthalsäureanhydrid, Trimellitsäureanhydrid, Maleinsäureanhydrid und Bernsteinsäureanhydrid ausgewählt, ist, bezogen auf das Gesamtgewicht des Polykondensats.

2. Zusammensetzung nach Anspruch 1, bei der das Polyol 3 bis 4 Hydroxygruppen aufweist.

3. Zusammensetzung nach einem der vorhergehenden Ansprüche, bei der das Polyol eine lineare oder verzweigte, gesättigte Kohlenwasserstoffverbindung mit 4 bis 10 Kohlenstoffatomen und 3 bis 6 Hydroxygruppen (OH) ist.

4. Zusammensetzung nach einem der vorhergehenden Ansprüche, bei der das Polyol unter Glycerol, Pentaerythritol, Sorbit und deren Gemischen und besonders Pentaerythritol ausgewählt ist.

5. Zusammensetzung nach einem der vorhergehenden Ansprüche, bei der das Polyol oder Polyolgemisch 16 bis 28 Gew.-% und besser 18 bis 25 Gew.-% des Gesamtgewichts des Polykondensats ausmacht.

6. Zusammensetzung nach einem der vorhergehenden Ansprüche, wobei die nichtaromatische Monocarbonsäure die Formel RCOOH aufweist, worin R eine lineare, verzweigte und/oder cyclische, gesättigte Kohlenwasserstoffgruppe mit 7 bis 27 Kohlenstoffatomen, besser 9 bis 19 Kohlenstoffatomen und noch besser 11 bis 17 Kohlenstoffatomen ist.

7. Zusammensetzung nach einem der vorhergehenden Ansprüche, wobei die Gruppe R linear oder verzweigt vorliegt und vorzugsweise 5 bis 31 Kohlenstoffatome ausweist.

8. nach einem der vorhergehenden Ansprüche, wobei die nichtaromatische Monocarbonsäure einzeln oder in Form eines Gemisches ausgewählt ist unter: Capronsäure, Caprylsäure, Isoheptansäure, 4-Ethylpentansäure, 2-Ethylhexansäure, 4,5-Dimethylhexansäure, 2-Heptylheptansäure, 3,5,5-Trimethylhexansäure, Octansäure, Isooctansäure, Nonansäure, Decansäure, Isononansäure, Laurinsäure, Tridecansäure, Myristinsäure, Palmitinsäure, Stearinsäure, Isostearinsäure, Arachinsäure, Behensäure, Cerotinsäure (Hexacosansäure); Cyclopentancarbonsäure, Cyclopentanessigsäure, 3-Cyclopentylpropionsäure, Cyclohexancarbonsäure, Cyclohexylessigsäure, 4-Cyclohexylbuttersäure.

9. Zusammensetzung nach einem der vorhergehenden Ansprüche, wobei die nichtaromatische Monocarbonsäure unter 2-Ethylhexansäure, Isooctansäure, Laurinsäure, Palmitinsäure, Isostearinsäure und deren Gemischen und insbesondere nur unter Isostearinsäure ausgewählt ist.

10. Zusammensetzung nach einem der vorhergehenden Ansprüche, wobei die nichtaromatische Monocarbonsäure oder das Gemisch dieser Säuren 8 bis 38 Gew.-% und besser 10 bis 35 Gew.-% des Gesamtgewichts des Polykondensats ausmacht.

11. Zusammensetzung nach einem der vorhergehenden Ansprüche, wobei die aromatische Monocarbonsäure einzeln oder in Form eines Gemisches ausgewählt ist unter: Benzoesäure, o-Toluylsäure, m-Toluylsäure, p-Toluylsäure, 1-Naphthoesäure, 2-Naphthoesäure, 4-tert-Butyl-benzosäure, 1-Methyl-2-naphthoesäure, 2-Isopropyl-1-Naphthoesäure.

12. Zusammensetzung nach einem der vorhergehenden Ansprüche, wobei die aromatische Monocarbonsäure einzeln oder in Form eines Gemisches unter Benzoesäure, o-Toluylsäure, m-Toluylsäure, 1-Naphthoesäure und deren Gemischen und besser Benzoesäure alleine ausgewählt ist.

13. Zusammensetzung nach einem der vorhergehenden Ansprüche, wobei die aromatische Monocarbonsäure oder das Gemisch dieser Säuren 20 bis 52 Gew.-%, insbesondere 22 bis 52 Gew.-% und besser 25 bis 50 Gew.-% des Gesamtgewichts des Polykondensats ausmacht.

14. Zusammensetzung nach einem der vorhergehenden Ansprüche, wobei die Polycarbonsäure oder ihr Anhydrid einzeln oder in Form eines Gemisches ausgewählt ist unter:
- Dicarbonsäuren, wie Decandisäure, Dodecandisäure, Cyclopropandicarbonsäure, Cyclohexandicarbonsäure, Cyclobutandicarbonsäure, Naphthalin-1,4-dicarbonsäure, Naphthalin-2,3-dicarbonsäure, Naphthalin-2,6-dicarbonsäure, Suberinsäure, Oxalsäure, Malonsäure, Bernsteinsäure, Phthalsäure, Terephthalsäure, Isophthalsäure, Pimelinsäure, Sebacinsäure, Azelainsäure, Glutarsäure, Adipinsäure, Fumarsäure, Maleinsäure;
- Tricarbonsäuren, wie Cyclohexantricarbonsäure, Trimellitsäure, 1,2,3-Benzoltricarbonsäure, 1,3,5-Benzoltricarbonsäure;
- Tetracarbonsäuren, wie Butantetracarbonsäure, Pyromellitsäure;
- cyclischen Anhydriden dieser Säuren und insbesondere Phthalsäureanhydrid, Trimellitsäureanhydrid, Maleinsäureanhydrid und Bernsteinsäureanhydrid.

15. nach einem der vorhergehenden Ansprüche, wobei die Polycarbonsäure oder ihr Anhydrid unter Phthalsäureanhydrid und/oder Isophthalsäure und besser nur Isophthalsäure ausgewählt ist.

16. Zusammensetzung nach einem der vorhergehenden Ansprüche, wobei die Polycarbonsäure und/oder ihr cyclisches Anhydrid 11 bis 22 Gew.-% und besser 12 bis 20 Gew.-% des Gesamtgewichts des Polykondensats ausmachen.

17. Zusammensetzung nach einem der vorhergehenden Ansprüche, bei der das Polykondensat ferner mindestens ein Silicon mit Hydroxyfunktion (OH) und/oder Carboxyfunktion (COOH) umfasst.

18. nach Anspruch 17, wobei das Silicon eine gewichtsmittlere Molmasse (Mw) im Bereich von 300 bit 20000, insbesondere 400 bis 10000 und noch besser 800 bis 4000 aufweist.

19. Zusammensetzung nach einem der Ansprüche 17 bis 18, wobei das Silicon die folgende Formel aufweist: 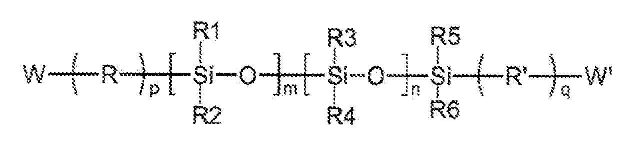 in der Formel:
- W und W' bedeuten unabhängig voneinander OH oder COOH, vorzugsweise W = W';
- p und q bedeuten unabhängig voneinander 0 oder 1;
- R und R' bedeuten unabhängig voneinander eine gesättigte oder ungesättigte, sogar aromatische, lineare, verzweigte und/oder cyclische, zweiwertige Gruppe auf Kohlenstoffbasis und insbesondere Kohlenwasserstoffbasis, die 1 bis 12 Kohlenstoffatome und insbesondere 2 bis 8 Kohlenstoffatome aufweist und gegebenenfalls ferner 1 oder mehrere Heteroatome enthält, die unter O, S und N und insbesondere O (Ether) ausgewählt sind; wobei R und/oder R' insbesondere die Formel-(CH₂)ₐ- mit a = 1-12 ausweisen können, insbesondere Methylen, Ethylen, Propylen, Phenylen:
oder die Formel -[(CH₂)ₓO]_{z}- mit x = 1, 2 oder 3 und z = 1-10; insbesondere x = 2 oder 3 und z = 1-4; besser x = 3 und z = 1;
- R₁ bis R₆ bedeuten unabhängig voneinander eine gesättigte oder ungesättigte, sogar aromatische, lineare, verzweigt und/oder cyclische, zweiwertige Gruppe auf Kohlenstoffbasis, die 1 bis 20 Kohlenstoffatome und insbesondere 2 bis 12 Kohlenstoffatome ausweist, wobei R₁ bis R₆ vorzugsweise gesättigt oder auch aromatisch sind und wobei sie insbesondere unter den Alkylgruppen und besonders den Gruppen Methyl, Ethyl, Propyl, Isopropyl, Butyl, Pentyl, Hexyl, Octyl, Decyl, Dodecyl und Octadecyl, den Cycloalkylgruppen und insbesondere Cyclohexyl, den Arylgruppen, insbesondere Phenyl und Naphthyl, den Arylalkylgruppen, insbesondere Benzyl und Phenethyl, sowie den Gruppen Tolyl und Xylyl ausgewählt werden können;
- m und n bedeuten unabhängig voneinander ganze Zahlen im Bereich von 1 bis 140 und sind so gewählt, dass die gewichtsmittlere Molmasse (Mw) des Silicons im Bereich von 300 bis 20000, insbesondere 400 bis 10000 und noch besser 800 bis 4000 liegt.

20. Zusammensetzung nach einem der Ansprüche 17 bis 19, wobei das Silicon einzeln oder in Form eines Gemisches unter den α,ω-Diol-polyalkylsiloxanen oder α,ω-Dicarboxy-polyalkylsiloxanen und insbesondere α,ω-Diol-polydimethylsiloxanen oder α,ω-Dicarboxy-polydimethylsiloxanen; α,ω-Diol-polyarylsiloxanen oder α,ω-Dicarboxy-polyarylsiloxanen und insbesondere α,ω-Diol-polyphenylsiloxanen oder α,ω-Dicarboxy-polyphenylsiloxanen; Polyarylsiloxanen mit Silanolfunktionen, wie Polyphenylsiloxan; Polyalkylsiloxanen mit Silanolfunktionen, wie Polydimethylsiloxan; Polyaryl/ alkylsiloxanen mit Silanolfunktionen, wie Polyphenyl/methylsiloxan oder Polyphenyl/propylsiloxan, ausgewählt, ist.

21. Zusammensetzung nach einem der Anspruche 17 bis 20, wobei das Silicon unter den α,ω-Diol-Polydimethylsiloxanen mit einer gewichtsmittleren Molmasse (Mw) im Bereich von 400 bis 10000, insbesondere im Bereich von 500 bis 5000 und noch besser im Bereich von 800 bis 4000 ausgewählt ist.

22. Zusammensetzung nach einem der Ansprüche 17 bis 21, wobei das Silicon 0,1 bis 15 Gew.-%, insbesondere 1 bis 10 Gew.-% und besser 2 bis 8 Gew.-% des Gesamtgewichts des Polykondensats ausmacht.

23. Zusammensetzung nach einem der vorhergehenden Ansprüche, wobei das Verhältnis Mol aromatische Monocarbonsäure / Mol nichtaromatische Monocarbonsäure im Bereich von 1,2 bis 8, insbesondere im Bereich von 1,3 bis 7,8, besonders im Bereich von 1,4 bit 7,5 und noch besser im Bereich von 1,9 bis 7 liegt.

24. Zusammensetzung nach einem der vorhergehenden Ansprüche, wobei das Polykondensat erhältlich ist durch Umsetzung von:
- mindestens einem Polyol, das einzeln oder in Form eines Gemisches unter 1,2,6-Hexantriol, Trimethylolethan, Trimethylolpropan, Glycerol; Pentaerythritol, Erythritol, Diglycerol, Ditrimethylolpropan; Xylitol, Sorbitol, Mannitol, Dipentacrythritol und/oder Triglycerol ausgewählt ist; und das vorzugsweise in einem Mengenanteil von 15 bis 30 Gew.-%. insbesondere 16 bis 28 Gew.-% und besser 18 bis 25 Gew.-%, bezogen auf das Gesamtgewicht des fertigen Polykondensats, enthalten ist;
- mindestens einer nichtaromatischen Monocarbonsäure, die einzeln oder in Form eines Gemisches unter Capronsäure, Caprylsäure, Isoheptansäure, 4-Ethylpentansäure, 2-Ethylhexansaure,4,5-Dimethylhexansäure, 2-Heptylheptansäure,3,5,5-Trimethylhexansäure, Octansäure, Isooctansäure, Nonansäure, Decansäure, Isononansäure, Laurinsäure, Tridecansäure, Myristinsäure, Palmitinsäure, Stearinsäure, Isostearinsäure, Arachinsäure, Behensäure, Cerotinsäure (Hexacosansäure); Cyclopentancarbonsäure, Cyclopentanessigsäure, 3-Cyclopentylpropionsäure, Cyclohexancarbonsäure, Cyclohexylessigsäure, 4-Cyclohexylbuttersäure ausgewählt ist;
und die vorzugsweise in einem Mengenanteil von 5 bis 40 Gew.-%, insbesondere 8 bis 38 Gew.-% und besser 10 bis 35 Gew.-%, bezogen auf das Gesamtgewicht des fertigen Polykondensats, enthalten ist;
- mindestens einer aromatischen Monocarbonsäure, die einzeln oder in Form eines Gemisches unter Benzoesäure, o-Toluylsäure, m-Toluylsäure, p-Toluylsäure, 1-Naphthoesäure, 2-Naphthoesäure, 4-*tert*-Butyl-benzosäure, 1-Methyl-2-naphthoesäure, 2-Isopropyl-1-naphthoesäure ausgewählt ist; und die vorzugsweise in einem Mengenanteil von 10 bis 55 Gew.-%, insbesondere 20 bis 52 Gew.-% und besser 25 bis 50 Gew.-%, bezogen auf das Gesamtgewicht des fertigen Polykondensats, enthalten ist; und
- mindestens einer Polycarbonsäure oder einem ihrer Anhydride, die einzeln oder in Form eines Gemisches unter Decandisäure, Dodecandisäure, Cyclopropandicarbonsäure, Cyclohexandicarbonsäure, Cyclobutandicarbonsäure, Naphthalin-1,4-dicarbonsäure, Naphthalin-2,3-dicarbonsäure, Naphthalin-2,6-dicarbonsäure, Suberinsäure, Oxalsäure, Malonsäure, Bernsteinsäure, Phthalsäure, Terephthalsäure, Isophthalsäure, Pimelinsäure, Sebacinsäure, Azelainsäure, Glutarsäure, Adipinsäure, Fumarsäure, Maleinsäure; Cyclohexantricarbonsäure, Trimellitsäure, 1,2,3-Benzoltricarbonsäure, 1,3,5-Benzoltricarbonsäure, Butantetracarbonsäure, Pyromellitsäure, Phthalsäureanhydrid, Trimellitsäureanhydrid, Maleinsäureanhydrid und Bernsteinsäureanhydrid ausgewählt ist;
und die vorzugsweise in einem Mengenanteil von 10 bis 25 Gew.-%, insbesondere 11 bis 22 Gew.-% und besser 12 bis 20 Gew.-%, bezogen auf das Gesamtgewicht des fertigen Polykondensats, enthalten, ist.

25. Zusammensetzung nach einem der vorhergehenden Ansprüche, wobei das Polykondensat erhältlich ist durch Umsetzung von:
- mindestens einem Polyol, das einzeln oder in Form eines Gemisches unter Glycerol, Pentaerythritol, Sorbitol und deren Gemischen und besser nur unter Pentaerythritol ausgewählt ist und das in einem Mengenanteil von 15 bis 30 Gew.-%, insbesondere 16 bis 28 Gew.-% und besser 18 bis 25 Gew.-%), bezogen auf das Gesamtgewicht des fertigen Poiykoriciensats, enthalten, ist;
- mindestens einer nichtaromatischen Monocarbonsäure, die einzeln oder in Form eines Gemisches unter 2-Ethylhexansäure, Isooctansäure, Laurinsäure, Palmitinsäure, Isostearinsäure und deren Gemischen und besser nur unter Isostearinsäure ausgewählt ist; und die in einem Mengenanteil von 5 bis 40 Gew.-%, insbesondere 8 bis 38 Gew.-% und besser 10 bis 35 Ges.-%, bezogen auf das Gesamtgewicht des fertigen Polykondensats, enthalten ist;
- mindestens einer aromatischen Monocarbonsaure, die einzeln oder in Form eines Gemisches unter Benzoesäure, o-Toluylsäure, m-Toluylsäure, 1-Naphthoesäure und besser nur unter Benzoesäure ausgewählt, ist; und die in einem Mengenanteil von 10 bis 55 Gew.-%, insbesondere 20 bis 52 Gew.-% und besser 25 bis 50 Gew.-%, bezogen auf das Gesamtgewicht des fertigen Polykondensats, enthalten ist; und
- mindestens einer Polycarbonsäure oder einem ihrer Anhydride, die einzeln oder in Form eines Gemisches unter Phthalsäureanhydrid und Isophthalsäure und besser unter Isophthalsäure alleine ausgewählt ist; und die in einem Mengenanteil von 10 bis 25 Gew.-%, insbesondere 11 bis 22 Gew.-% und besser 12 bis 20 Gew.-%, bezogen auf das Gesamtgewicht des fertigen Polykondensats, enthalten ist.

26. Zusammensetzung nach einem der vorhergehenden Ansprüche, wobei das Polykondensat mindestens eine der folgenden Eigenschaften aufweist:
- einen in mg Kaliumhydroxid pro g Polykondensat ausgedrückten Säureindex von 8 oder darüber, insbesondere im Bereich von 8 bis 40 und noch besser im Bereich von 10 bis 30; und/oder
- einen in mg Kaliumhydroxid pro g Polykondensat ausgedrückten Hydroxyindex von 30 oder darüber, insbesondere im Bereich von 30 bis 100 und noch besser im Bereich von 40 bis 90;
- eine bei 110 °C gemessene Viskosität im Bereich von 75 bis 6000 mPa·s, insbesondere im Bereich von 80 bis 5500 mPa·s, besonders 90 bis 5000 mPa·s und noch besser 200 bis 4800 mPa·s;
- bei 25°C eine Löslichkeit in Butylacetat oder Ethylacetat in einer Menge von mindestens 50 Gew.-%;
- bei 25 °C eine Viskosität einer Lösung des Polymers in Butylacetat oder Ethylacetat in einer Konzentration von 70 Gew.-% im Bereich von 100 bis 1500 mPa·s und insbesondere im Bereich von 120 bis 900 mPa·s.

27. Zusammensetzung nach einem der vorhergehenden Ansprüche, wobei das Polykondensat in einem Mengenanteil im Bereich von 0,1 bis 70 Gew.-%, vorzugsweise 2 bis 50 Gew.-%, besonders 3 bis 35 Gew.-%, sogar 5 bis 20 Ges.-% und noch besser 6 bis 18 Gew.-%, bezogen auf das Gesamtgewicht der fertigen kosmetischen ober pharmazeutischen Zusammensetzung, vorliegt.

28. Zusammensetzung nach einem der vorhergehenden Ansprüche, wobei das kosmetisch oder pharmazeutisch akzeptable Medium mindestens eine Verbindung enthält, die unter Wasser, Alkoholen, Polyolen, Ketonen, Estern, Ethern, Alkanen, Aldehyden, Ölen auf Kohlenstoffbasis, Silikonölen, fluorierten Siliconölen und deren Gemischen ausgewählt ist.

29. Zusammensetzung nach einem der vorhergehenden Ansprüche, wobei das kosmetisch oder pharmazeutisch akzeptable Medium mindestens ein organisches Lösemittel enthält, das ausgewählt ist unter:
- bei Raumtemperatur (25 °C) flüssigen Ketonen, wie Methylethylketon, Methylisobutylketon, Diisobutylketon, Isophoron, Cyclohexanon, Aceton;
- bei Raumtemperatur flüssigen Alkoholen, wie Ethanol, Isopropanol, Diacetonalkohol, 2-Butoxyethanol, Cyclohexanol;
- bei Raumtemperatur flüssigen Propylenglycolethern, wie Propylenglycolmonomethylether, Propylenglycolmonomethyletheracetat, Dipropylenglycolmono-*n*-butylether;
- cyclischen Ethern, wie γ-Butyrolacton;
- kurzkettigen Estern (mit insgesamt 3 bis 8 Kohlenstoffatomen), wie Ethylacetat, Methylacetat, Propylacetat, Isopropylacetat, *n*-Butylacetat, Isopentylacetat, Methoxypropylacetat, Butyllactat;
- bei Raumtemperatur flüssigen Ethern, wie Diethylether, Dimethylether oder Dichlordiethylether;
- bei Raumtemperatur flüssigen. Alkanen, insbesondere mit 5 bis 12 Kohlenstoffatomen, wie Decan, Heptan, Dodecan, Isododecan, Cyclohexan;
- bei Raumtemperatur flüssigen Aldehyden, wie Benzaldehyd, Acetaldehyd; und
- deren Gemischen.

30. Zusammensetzung nach einem der vorhergehenden Ansprüche, wobei das kosmetisch oder pharmazeutisch akzeptable Medium mindestens ein Lösemittel enthält, das unter den kurzkettigen Estern mit 3 bis 8 Kohlenstoffatomen, wie Ethylacetat, Methylacetat, Propylacetat, Isopropylacetat, n-Butylacetat, Isopentylacetat, Methoxypropylacetat, Butyllactat; bei Raumtemperatur flüssigen Alkoholen, wie Ethanol, Isopropanol, Diacetonalkohol, 2-Butoxyethanol, Cyclohexanol; und deren Gemischen ausgewählt ist.

31. Zusammensetzung nach einem der vorhergehenden Ansprüche, wobei das kosmetisch oder pharmazeutisch akzeptable Medium ferner mindestens einen Bestandteil enthält, der unter den Ölen auf Kohlenstoffbasis, Ölen auf Kohlenwasserstoffbasis, fluorierten Ölen und/oder Siliconölen mineralischer, tierischer, pflanzlicher oder synthetischer Herkunft; filmbildenden Polymeren; Hilfsstoffen für die Filmbildung; Verdickungsmitteln; sekundären Harzen; Wachsen pflanzlicher, tierischer, mineralischer oder synthetischer Herkunft, wobei es sich such um Siliconwachse handeln kann; Farbmitteln; Antioxidantien, Parfums, etherischen Ölen, Konservierungsmitteln, kosmetischen Wirkstoffen, Hydratisierungsmitteln, Vitaminen, Ceramiden, Sonnenschutzfiltern, grenzflächenaktiven Stoffen, Spreitmitteln, Netzmitteln, Dispergiermitteln, Schaumverhütungsmitteln, Neutralisationsmitteln, Stabilisatoren und deren Gemischen ausgewählt ist.

32. Zusammensetzung nach einem der vorhergehenden Ansprüche, die enthält:
- einzeln oder als Gemisch 0,1 bis 50 Gew.-%, vorzugsweise 2 bis 35 Gew.-%, insbesondere 5 bis 20 Gew.-% und besser 6 bis 18 Gew.-% Polykondensat nach einem der vorhergehenden Ansprüche, bezogen auf das Gesamtgewicht der kosmetischen Zusammensetzung,
- 1 bis 70 Gew-.%, vorzugsweise 2 bis 60 Gew.-% und besser 5 bis 45 Gew.-%) filmbildendes Polymer, bezogen auf das Gesamtgewicht der kosmetischen Zusammensetzung, das insbesondere unter den Cellulosepolymeren, wie Nitrocellulose, Celluloseacetat, Celluloseacetobutyrat, Celluloseacetopropionat, Ethylcellulose; Polyurethanen, Acrylpolymeren, Vinylpolymeren, Polyvinylhutyralen, Alkydharzen, bei der Aldehyd-Kondensation gebildeten Harzen, wie Arylsulfonamid - Formaldeyhd-Harzen, beispielsweise das Toluolsulfonamid-Formaldehydharz, Arylsulfonamidepoxyharzen oder Ethyltosylamidharzen; Polymeren natürlicher Herkunft und deren Gemischen ausgewählt ist:
- 10 bis 95 Gew.-%, vorzugsweise 15 bis 80 Gew.-% und besser 20 bis 60 Ges.-% organisches Lösemittel, bezogen auf das Gesamtgewicht der kosmetischen Zusammensetzung, das insbesondere unter den bei Raumtemperatur flüssigen Ketonen; bei Raumtemperatur flüssigen Alkoholen, bei Raumtemperatur flüssigen Propylenglycolethern; cyclischen Ethern; kurzkettigen Estern (mit insgesamt 3 bis 8 Kohlenstoffatomen); bei Raumtemperatur flüssigen Ethern; bei Raumtemperatur flüssigen Alkanen; bei Raumtemperatur flüssigen Aldehyden; und deren Gemischen ausgewählt ist;
- gegebenenfalls mindestens ein Farbmittel, das in der Zusammensetzung in einer Menge von 0,01 bis 50 Gew.-%, bezogen auf das Gesamtgewicht der Zusammensetzung, vorzugsweise 0,1 bis 40 Ges.-% und besser 1 bis 30 Gew.-% enthalten ist.

33. Zusammensetzung nach einem der vorhergehenden Ansprüche, die als Produkt für die Pflege und/oder zum Schminken der Haut des Körpers oder des Gesichts, der Lippen, der Wimpern, der Augenbrauen, der Haare, der Kopfhaut, oder der Nägel; als Sonnenschutzprodukt oder Selbstbräunungsmittel; oder als Produkt für die Haarbehandlung vorliegt.

34. Zusammensetzung nach einem der vorhergehenden Ansprüche, die in Form eines Nagellacks vorliegt.

35. Verfahren zur kosmetischen Behandlung von Keratinsubstanzen, insbesondere der Haut des Körpers oder des Gesichts, der Nägel, der Haare und/oder der Wimpern, das das Auftragen einer wie in den Ansprüchen 1 bis 34 definierten Zusammensetzung auf die Keratinsubstanzen umfasst.

36. Kosmetisches Verfahren zum Schminken der Nägel, das das Auftragen einer wie in den Ansprüchen 1 bis 34 definierten Zusammensetzung auf die Nägel umfasst.

37. Polykondensat, erhältlich durch Umsetzung folgender Verbindungen:
- mindestens eines Polyols, das einzeln oder in Form eines Gemisches unter 1,2,6-Hexantriol, Trimethylolethan, Trimethylolpropan, Glycerol; Pentaerythritol, Erythritol, Diglycerol, Ditrimethylolpropan; Xylitol, Sorbitol, Mannitol, Dipentaerythritol und/oder Triglycerol ausgewählt ist; und das in einem Mengenanteil von 15 bis 30 Gew.-%, insbesondere 16 bis 28 Gew.-% und besser 18 bis 25 Gew.-%, bezogen auf das Gesamtgewicht des fertigen Polykondensats, enthalten ist;
- mindestens einer nichtaromatischen Monocarbonsäure, die einzeln oder in Form eines Gemisches unter Capronsäure, Caprylsäure, Isoheptansäure, 4-Ethylpentansäure, 2-Ethylhexansäure, 4,5-Dimethylhexansäure, 2 - Heptylheptansäure, 3,5,5-Trimethylhexansäure, Octansäure, Isooctansäure, Nonansäure, Decansäure, Isononansäure, Laurinsäure, Tridecansäure, Myristinsäure, Palmitinsäure, Stearinsäure, Isostearinsäure, Arachinsäure, Behensäure, Cerotinsäure (Hexacosansäure); Cyclopentancarbonsäure, Cyclopentanessigsäure, 3-Cyclopentylpropionsäure, Cyclohexancarbonsäure, Cyclohexylessigsäure, 4-Cyclohexylbuttersäure ausgewählt ist; und die in einem Mengenanteil von 5 bis 40 Gew.-%, insbesondere 8 bis 38 Gew.-% und besser 10 bis 35 Ges.-%, bezogen auf das Gesamtgewicht des fertigen Polykondensats, enthalten ist;
- mindestens einer aromatischen Monocarbonsäure, die einzeln oder in Form eines Gemisches unter Benzoesäure, o-Toluylsäure, m-Toluylsäure, p-Toluylsäure, 1-Naphthoesäure, 2-Naphthoesäure, 4-*tert*-Butyl-benzosäure, 1-Methyl-2-naphthoesäure, 2-Isopropyl-1-naphthoesäure ausgewählt ist und die in einem Mengenanteil von 10 bis 55 Gew.-%, insbesondere 20 bis 52 Gew.-% und besser 25 bis 50 Gew.-%, bezogen auf das Gesamtgewicht des fertigen Polykondensats, enthalten ist; und
- mindestens einer Polycarbonsäure oder einem ihrer Anhydride, die einzeln oder in Form eines Gemisches unter Decandisäure, Dodecandisäure, Cyclopropandicarbonsäure, Cyclohexandicarbonsäure, Cyclobutandicarbonsäure, Naphthalin-1,4-dicarbonsaure, Naphthalin-2,3-dicarbonsäure, Naphthalin-2,6-dicarbonsäure, Suberinsäure, Oxalsäure, Malonsäure, Bernsteinsäure, Phthalsäure, Terephthalsäure, Isophthalsäure, Pimelinsäure, Sebacinsäure, Azelainsäure, Glutarsäure, Adipinsäure, Fumarsäure, Maleinsäure; Cyclohexantricarbonsaure, Trimellitsäure, 1,2,3-Benzoltricarbonsäure, 1,3,5-Benzoltricarbonsäure, Butantetracarbonsäure, Pyromellitsäure, Phthalsäureanhydrid, Trimellitsäureanhydrid; Maleinsäureanhydrid und Bernsteinsäureanhydrid ausgewählt ist, und die in einem Mengenanteil von 10 bis 25 Gcw.-%, insbesondere 11 bis 22 Gew.-% und besser 12 bis 20 Gew.-%; bezogen auf das Gesamtgewicht des fertigen Polykondensats, enthalten ist.

38. Polykondensat nach Anspruch 37, wobei das Polykondensat ferner mindestens ein Silicon mit Hydroxyfunktion (OH) und/oder Carboxyfunktion (COOH) umfasst.

39. Polykondensat nach einem der Ansprüche 37 bis 38, wobei das Silicon die folgende Formel aufweist: 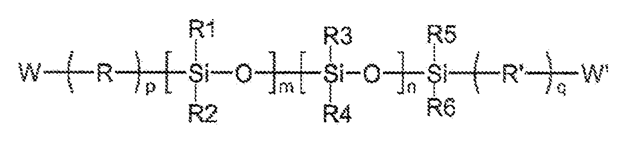 in der Formel:
- W und W' bedeuten unabhängig voneinander OH oder COOH, vorzugsweise W = W';
- p und q bedeuten unabhängig voneinander 0 oder 1;
- R und R' bedeuten unabhängig voneinander eine gesättigte oder ungesättigte, sogar aromatische, lineare, verzweigte und/oder cyclische, zweiwertige Gruppe auf Kohlenstoffbasis und insbesondere Kohlenwasserstoffbasis, die 1 bis 12 Kohlenstoffatome und insbesondere 2 bis 8 Kohlenstoffatome ausweist und gegebenenfalls ferner 1 oder mehrere Heteroatome enthält, die unter O, S und N und insbesondere O (Ether) ausgewählt sind;
wobei R und/oder R' die Formel - (CH₂)ₐ- mit a = 1-12 aufweisen können, insbesondere Methylen, Ethylen, Propylen, Phenylen:
oder die Formel -[(CH₂)ₓO]_{z}- mit x = 1, 2 oder 3 und z = 1 - 10; insbesondere x = 2 oder 3 und z = 1-4; besser x = 3 und z = 1;
- R₁ bis R₆ bedeuten unabhängig voneinander eine gesättigte oder ungesättigte, sogar aromatische, lineare, verzweigte und/oder cyclische, zweiwertige Gruppe auf Kohlenstoffbasis, die 1 bis 20 Kohlenstoffatome und insbesondere 2 bis 12 Koblenstoffatome aufweist, wobei R₁ bis R₆ vorzugsweise gesättigt oder auch aromatisch sind und wobei sie insbesondere unter den Alkylgruppen und besonders den Gruppen Methyl, Ethyl, Propyl, Isopropyl, Butyl, Pentyl, Hexyl, Octyl, Decyl, Dodecyl und Octadecyl, den Cycloalkylgruppen und insbesondere Cyclohexyl, den Arylgruppen, insbesondere Phenyl und Naphthyl, den Arylalkylgruppen, insbesondere Benzyl und Phenethyl, sowie den Gruppen Tolyl und Xylyl ausgewählt werden können; m und n bedeuten unabhängig voneinander ganze Zahlen im Bereich von 1 bis 140 und sind so gewählt, dass die gewichtsmittlere Molmasse (Mw) des Silicons im Bereich von 300 bis 20000, insbesondere 400 bis 10000 und noch besser 800 bis 4000 liegt.

40. Polykondensat nach einem der Ansprüche 37 bis 39, wobei das Silicon einzeln oder in Form eines Gemisches unter den α,ω-Diol-polyalkylsiloxanen oder α,ω-Dicarboxy-polyalkylsiloxanen und insbesondere α,ω-Diol-polydimethylsiloxanen oder α,ω-Dicarboxy-polydimethylsiloxanen, α,ω-Diol-polyarylsiloxanen oder α,ω-Dicarboxy-polyarylsiloxanen und insbesondere α,ω-Diol-Polyphenylsiloxanen oder α,ω-Dicarboxy-polyphenylsiloxanen; Polyarylsiloxanen mit Silanolfunktionen, wie Polyphenylsiloxan; Polyalkylsiloxanen mit Silanolfunktionen, wie Polydimethylsiloxan; Polyaryl/alkylsiloxanen mit Silanolfunktionen, wie Polyphenyl/methylsiloxan oder Polyphenyl / propylsiloxan, ausgewählt ist.

41. Polykondensat nach einem der Ansprüche 37 bis 40, wobei das Silicon 0,1 bis 15 Gew.-%, insbesondere 1 bis 10 Gew.-% und besser 2 bis 8 Gew.-% des Gesamtgewichts des Polykondensats ausmacht.

42. Verfahren zur Herstellung von Polykondensaten nach einem der Ansprüche 37 bis 41, das darin besteht:
- das Polyol und die aromatischen und nichtaromatischen Monocarbonsäuren werden, vermischt;
- das Gemisch wird unter einer inerten Atmosphäre zunächst bis zur Schmelztemperatur (im Allgemeinen 100 - 130 °C) und anschließend auf eine Temperatur im Bereich von 150 bis 220 °C erwärmt, bis die Monocarbonsäuren vollständig verbraucht sind, dann
- das Gemisch wird gegebenenfalls auf eine Temperatur im Bereich von 90 bis 150 °C abgekühlt,
- die Polycarbonsäure und/oder das cyclische Anhydrid und optional das Silicon mit Hydroxy- oder Carboxyfunktionen werden zugegeben, dann
- man erwärmt nachmals auf eine Temperatur von 220 °C oder darunter.

43. Verfahren nach Anspruch 43, bei dem ein oder mehrere Antioxidationsmittel insbesondere in einer Konzentration im Bereich von 0,01 bis 1 Ges.-%, bezogen auf das Gesamtgewicht der Monomere, in das Reaktionsmedium gegeben werde.

44. Verfahren nach Anspruch 43, wobei das Antioxidationsmittel ausgewählt ist unter: BHT, BHA, TBHQ, 1,3,5-Trimethyl-2,4,6,tris(3,5-di-*tert*-butyl-4-hydroxybenzyl)-benzol, Octadecyl-3,5-di-*tert*-butyl-4-hydroxycinnamat, Tetrakis-methylen-3-(3,5-di-*tert*-butyl-4-hydroxy-phenyl)propionat-methan, Octadecyl-3-(3,5-di-*tert*-butyl-4-hydroxyphenyl)propionat-2,5-di-*tert*-butyl-hydrochinon, 2,2-Methyl-bis-(4-methyl-6-*tert*-butyl-phenol), 2,-Methylen-bis(4-ethyl-6-*tert*-butyl-phenol), 4,4-Butyliden-bis(6-*tert*-butyl-m-kresol), N,N-Hexaulethylen-bis(3,5-di-*tert*-butyl-4-hydroxyhydrocinnamamid), Pentaerythritol-tetrakis-(3-(3,5-di-*tert*-butyl-4-hydroxyphenyl)propionat), Octadecyl 3-(3,5-di-*tert-*butyl-4-hydroxphenyl)-propionat; 1,3,5-Tris(3,5-di-*tert*-butyl-4-hydroxybenzyl) -1,3,5-triazin-2,4,6-(1H, 3H, 5H)-trion;
Di(stearyl)pentacrythritol-diphosphit, Tris(2,4-di-*tert*-butyl; phenyl)phosphit; Dilauryl-thiodipropionat; Bis(2,4-di-*tert-*butyl)pentaerythritol-diphosphit; Bis(2,4-bis[2-phenylpropan-2-yl]phenyl)pentaerythritol~diphosphit, Triphenylphosphit, (2,4-Di-*tert*-butylphenyl)phentaerythritol-diphosphit; Tris(nonylphenyl)-phosphit; 1: 1-Gemisch von N,N Hexamethylen-bis(3,5-di-*tert-*butyl-4-hydroxy-hydrocinnamamid) und Tris(2,4-di-*tert-*butylphenyl)phosphat; Tetrakis-(2,4-di-*tert*-butylphenyl)phosphit; Distearylthiodipropionat; 2,4-Bis(octylthiomethyl)-o-kresol; 4,6-Bis(dodecythiomethyl)-o-kresol.
